Europäisches Patentamt

European Patent Office

Office européen des brevets

(11) Publication number: **0 319 944 B1**

## EUROPEAN PATENT SPECIFICATION

(45) Date of publication of patent specification: **21.09.94**

(51) Int. Cl.⁵: **C12N 15/00**, C12P 21/02, C12N 5/00

(21) Application number: **88120423.4**

(22) Date of filing: **07.12.88**

(54) **Co-expression in eukaryotic cells.**

(30) Priority: **08.12.87 US 130370**
**15.01.88 US 144357**

(43) Date of publication of application:
**14.06.89 Bulletin 89/24**

(45) Publication of the grant of the patent:
**21.09.94 Bulletin 94/38**

(84) Designated Contracting States:
**AT BE CH DE ES FR GB GR IT LI LU NL SE**

(56) References cited:
EP-A- 0 072 925     EP-A- 0 253 455
WO-A-85/01959     WO-A-87/04187
WO-A-88/05825     WO-A-88/08035

**Kaufman et al, Mol. Cell. Biol. vol.9 (1989)
pp.1233-1242.**

(73) Proprietor: **ZYMOGENETICS, INC.**
**4225 Roosevelt Way N.E.
Seattle, WA 98108 (US)**

(72) Inventor: **Mulvihill, Eileen R.**
**4016 Francis Avenue North
Seattle Washington 98103 (US)**
Inventor: **Berkner, Kathleen L.**
**3032-22nd Avenue West
Seattle Washington 98199 (US)**
Inventor: **Foster, Donald C.**
**4908 N.E. 97th
Seattle Washington 98102 (US)**
Inventor: **Kumar, Ashok A.**
**4509 Fremont Avenue No. 4
Seattle Washington 98103 (US)**
Inventor: **Mackay, Vivian L.**
**120 N.E. 51st
Seattle Washington 98105 (US)**
Inventor: **Parker, Gary E.**
**170 South Meadowlark Road
Coupeville Washington 98239 (US)**

(74) Representative: **Brown, John David et al
FORRESTER & BOEHMERT
Franz-Joseph-Strasse 38
D-80801 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.09/3.3.3)

## Description

Technical Field

The present invention relates generally to the production of proteins, and more particularly, to the production of proteins in biologically active form and in economically feasible amounts.

Background of the Invention

Recombinant DNA technology has been used to produce a variety of proteins of therapeutic or other economic value, including enzymes, growth factors and peptide hormones. These proteins have been produced in bacteria, fungal cells and, more recently, cultured mammalian cells. Because single-celled organisms cannot correctly process many human proteins, it has often been necessary to use cultured mammalian cells to make these proteins.

Mammalian cells can be transfected to express cloned DNA by well-established laboratory procedures. However, not all mammalian cell types will efficiently express transfected DNA sequences, and cells which have been shown to be efficient expressors of one transfected sequence will in other cases produce only low levels of different gene products. Low expression levels for transfected genes may result from degradation of the protein product intra and/or extracellularly, production of inactive form(s) of the protein or production of form(s) of the protein that are cytotoxic. Low levels of protein or activity may also result from an unstable mRNA sequence, proteolytic activation or inadequate, inefficient or improper processing by the host cell. Processing steps which may be necessary for the activity or secretion of a newly synthesized protein include specific proteolytic cleavage, subunit polymerization, disulfide bond formation, post-translational or co-translational modification of certain amino acids and glycosylation.

These problems in protein production reflect the specialized nature of cells derived from higher organisms. Mammalian cells derived from a particular tissue may not properly produce a protein that is not normally made by that tissue. In addition, mammalian cell lines adapted to grow in culture are derived from tumors or are otherwise abnormal, often leading to unpredictable protein processing.

For example, a number of research groups have produced human coagulation factor IX in cultured mammalian cells (Kaufman et al., J. Biol. Chem. 261:9622-9628, 1986; Anson et al., Nature 315:683-685, 1985; Hagen et al., EP 200,421; Busby et al., Nature 316:271-273, 1985). Despite efforts to maximize production of biologically active protein through the use of strong promoters, enhancers, increased gene copy number, etc., and despite the relatively high levels of factor IX mRNA observed, levels of active factor IX produced by these transfected cell lines do not exceed about 5 ug/ml of cell culture medium. In some cases precursor forms of factor IX are made but mature protein is ineffectively secreted from the host cell.

Problems with protein production have previously been dealt with by experimenting with a number of different cell types and by selecting and screening a large number of isolates of a particular transformed or transfected strain or cell line. Such an approach is extremely labor intensive and carries no assurance of success. Consequently, there is a need in the art for a method of systematically and predictably producing recombinant cells which can express proteins of interest in an active form in economically feasible amounts. The present invention provides such a system, and further provides other related advantages.

Disclosure of Invention

Briefly stated, the present invention provides a method of producing a protein of interest comprising the steps of (a) introducing into a eukaryotic host cell a first DNA sequence encoding the protein of interest and at least one additional DNA sequence, the additional DNA sequence encoding a protein which processes or stabilizes the protein of interest; (b) culturing the host cell under conditions which allow the first DNA sequence and the additional DNA sequence(s) to be expressed; and (c) isolating the protein of interest from the host cell. The step of introducing the DNA sequences into the host cell may be through (a) cotransfection or cotransformation with multiple vectors, each containing a separate expression unit; or (b) transfection or transformation with a single vector containing multiple expression units. When the host cell is a mammalian host cell, the step of introducing may also be through transfection with a single vector containing a single expression unit that is transcribed into a polycistronic message. When yeast host cells are utilized, a preferred method for introducing the DNA sequences comprises (a) transforming the yeast host cell with a single expression unit containing the additional DNA sequence(s); (b) isolating host cells which stably produce the processing or stabilizing activity; and (c) transforming the isolated host cells with the first DNA sequence encoding the protein of interest. Preferably, the initial transforming step results in the integration of the single expression unit into the yeast host cell genome.

Preferred first DNA sequences include those encoding plasma serine proteases such as t-PA, factor VII, factor IX, factor X, protein C and plasminogen. Preferred additional DNA sequences include those encoding proteases, protease inhibitors, gamma-carboxylase, and proteins which bind to the protein of interest.

In another aspect of the present invention, eukaryotic host cells into which a DNA sequence encoding a protein of interest as described above and one or more additional DNA sequences encoding a protein or proteins which process or stabilize the protein of interest as described above have been introduced are disclosed. Preferred eukaryotic host cells include mammalian host cells and yeast host cells.

Brief Description of the Drawings

Figure 1 illustrates the construction of the plasmid Zem99.

Figure 2 illustrates the construction of the t-PA expression vector Zem219.

Figure 3 illustrates the construction of Zem228.

Figure 4 illustrates the subcloning of the alpha-1-antitrypsin cDNA.

Figure 5 illustrates the construction of expression vector Zem235.

Figure 6 illustrates the nucleotide sequence of a plasminogen cDNA, together with the encoded amino acid sequence.

Figure 7 illustrates the production of plasminogen in transfected BHK cells (a), and in transfected BHK cells co-expressing alpha-1-antitrypsin (b). [Lanes 1-media samples; lanes 2-cytoplasmic extracts. Arrows indicate the position of intact plasminogen (92 kd)].

Figure 8 illustrates the construction of the vector pD5. Symbols used are: 0-1, the adenovirus 5 0-1 map unit sequence; E, the SV40 enhancer; MLP, the adenovirus 2 major late promoter; L1-3, the adenovirus 2 tripartite leader; 5′, 5′ splice site; 3′, 3′ splice site; p(A), polyadenylation signal; DHFR, dihydrofolate reductase gene.

Figure 9 illustrates the construction of the vector pDX. Symbols are used as set forth for Figure 8.

Figure 10 illustrates the construction of expression vectors containing the S. cerevisiae KEX2 gene.

Figure 11 illustrates the results of an assay for activated protein C on media samples from transfected 293 cells.

Figure 12 illustrates the anticoagulant activity of activated protein C prepared in transfected mammalian cells.

Figure 13 shows the results of a radioimmune precipitation of factor VII produced by cells co-expressing factor IX (lane 1) and by factor VII-transfected control cells (lane 2). The arrow indicates the position of single-chain factor VII.

Figure 14 illustrates the construction of a yeast expression vector containing a DNA sequence encoding a thrombin-cleavable fusion protein. "MI-3" indicates the insulin precursor DNA sequence.

Figure 15 illustrates the nucleotide sequence of a synthetic aprotinin gene.

Best Mode for Carrying Out the Invention

Prior to setting forth the invention, it may be helpful to an understanding thereof to set forth definitions of certain terms to be used hereinafter.

Stabilize: The term stabilize is used herein to denote the protection of a protein from degradation. Stabilization may proceed by a number of mechanisms, including inhibition of a proteolytic enzyme which would otherwise degrade the protein of interest, binding to the protein of interest to protect it from a proteolytic enzyme, and binding to or otherwise inhibiting the action of a cofactor or other molecule required for the activity of a protease.

Process: As used herein, "process" means to modify the structure of a protein. Processing of proteins includes such modifications as specific proteolytic cleavage to produce a multi-chain protein or remove peptides from protein precursors; modification of amino acids, including carboxylation and hydroxylation; and carbohydrate addition at specific sites. Processing may be necessary for full biological activity of a protein or may be required to enable the secretion of a protein from a cell.

Transfection and Transformation: The process of introducing cloned DNA into host cells. Transfection refers to inserting DNA into mammalian cell lines. The process of inserting DNA into fungal and bacterial cells is known as transformation. A number of transfection and transformation procedures are known in the art.

As noted above, cells containing cloned DNA sequences do not always produce proteins encoded by those cloned sequences at economically feasible levels or in biologically active form. This is often due to the origin of the cell or its abnormal nature. In many instances it may not be possible to obtain a cultured cell line or host cell strain with the necessary characteristics to enable it to produce a particular protein at the desired level. In any event, screening a large number of potential host cell lines may not be feasible and carries no assurance of success.

The present invention overcomes the shortcomings of available cells by providing a method of introducing into a eukaryotic host cell a gene or cDNA encoding a protein of interest together with one or more additional DNA sequences encoding a protein or proteins which process or stabilize the protein of interest. Processing proteins include proteases which cleave a precursor protein at a particular site to provide the mature and/or active form of the protein, for example peptidases which remove signal peptides or propeptides, or which cleave a single-chain polypeptide to a multi-chain form. Other examples of processing proteins are those which modify amino acids, such as gamma-carboxylase, an enzyme which modifies specific glutamic acid residues of certain clotting factors and other calcium binding proteins. Other processing proteins include enzymes responsible for the conversion of aspartic acid to $\beta$-hydroxy aspartic acid, a modification necessary for the activity of protein C; those responsible for the addition of carbohydrate chains to glycoproteins; and those responsible for myristoylation, C-terminal amino acid removal, hydroxylation of proline residues, sulfation and C-terminal amidation. Stabilizing proteins include protease inhibitors which block the proteolytic degradation of the protein of interest; proteins which bind to the protein of interest making it unavailable as a substrate; proteins which bind to protein co-factors, ions, or other molecules required by a protease; and proteins which inactivate co-factors. It will be appreciated that a particular eukaryotic host cell can be transfected or transformed to produce several processing proteins, several stabilizing proteins, or a combination of stabilizing and processing proteins.

The present invention is based, in part, on the unexpected discovery that a variety of processing proteins will function outside their native environments. For example, it is disclosed herein that factor IX, an enzyme which normally functions in the blood, can activate factor VII intracellularly. Additionally, it has been found that the product of the yeast KEX2 gene functions normally within mammalian cells. The observed processing indicates that both the protein of interest and the processing protein are unexpectedly targeted to the same cellular compartment. The observed function of these processing proteins is also surprising in view of the fact that the proteins of interest may not be produced in large amounts or in an intact or active form in a recombinant cell.

By characterizing a naturally occurring protein of interest and, as necessary, the gene or cDNA encoding it, one can deduce the nature of the processing steps involved in its biosynthesis. Characterization of the recombinant form of the protein will reveal whether or not it has been correctly processed and, if not, what processing steps were omitted. According to the present invention, proper processing is provided through supplying the missing activity or augmenting a limiting activity. This activity may be supplied as the protein which normally processes the protein of interest, or as a related protein which normally performs a similar function in another context, such as a protein from a different cell type. An example of the latter case is the use of the yeast KEX2 gene to supply the processing protein which cleaves protein C or an activated protein C precursor to the two-chain form. Blocks in the secretion of a protein of interest can be determined by characterizing the intracellular and secreted forms of the recombinant protein. In this way, the missing or limiting processing step is determined.

In some instances one will know the identity of the protein supplying the missing activity. In this case, the desired gene or cDNA is cloned and introduced into the chosen host cell.

When the protein responsible for the missing activity is not known, the protein of interest is analyzed, the nature of the missing processing step is determined, and a protein known to perform that function is selected. A suitable protein may be selected from known and available DNA sequences encoding proteins with similar activity. Many such processing proteins have been characterized. For example, Kettner and Shaw (Meth. in Enzymology 80:826-842, 1981) have characterized the specificities of a number of proteases. Alternatively, a DNA sequence encoding the needed processing protein may be identified by transfecting cells with a mammalian expression library and selecting those that exhibit the needed activity.

Where protein stabilization is desired, cells transfected or transformed to produce the protein of interest are grown in the presence of various stabilizing proteins and assayed for production of the intact protein of interest. Generally, the cells will be labeled with a radioisotope and proteins will be analyzed by radioimmune precipitation and gel electrophoresis, or by other conventional techniques. The presence of the intact protein of interest in the culture medium indicates that the stabilizing protein is protecting the protein of interest from degradation. Stabilization may also lead to phenotypic changes in the host cell. For example, protease activity may cause cell detachment, which may be corrected by inhibiting that activity. Correction is indicated by the presence of the normal (adherent) phenotype.

When the desired processing or stabilizing protein has been identified, the DNA sequence encoding it and the DNA sequence encoding the protein of interest are introduced into chosen host cells as described below. Cells expressing the introduced sequences are selected and screened for production of the protein of interest in the desired form or at the desired level. Cells which satisfy these criteria are then cloned and scaled up for production.

Proteins of interest which can be produced using the methods of the present invention include a variety of plasma serine proteases, such as coagulation factors VII, IX and X, activated factor VII (designated factor VIIa), activated factor X (factor Xa), protein C, activated protein C, protein S, tissue plasminogen activator (t-PA), plasminogen, and analogs and derivatives of these proteins, although virtually any protein of interest could be produced. The methods are particularly suited to the production of these serine proteases due to the post-translational processing necessary for their activity and/or secretion by the host cell.

According to the present invention, coagulation factors requiring gamma-carboxylation of specific glutamic acid residues for their biological activity may be secreted at high levels by cells into which has been introduced a DNA sequence encoding gamma-carboxylase. The gamma-carboxylation step has been found by the inventors to be limiting in mammalian cell lines commonly used for the production of recombinant coagulation factors. Furthermore, the invention may permit the production of biologically active gamma-carboxylated proteins in non-mammalian host cells such as yeast cells.

Coagulation factor VII may be made in activated form by transfecting cells with DNA sequences encoding factor VII and factor IX. The precursor form of factor VII is activated by the factor IX and factor VIIa is secreted by the cells. Alternatively, a protein having the biological activity of factor VIIa may be produced by co-expressing a DNA sequence encoding a derivative or analog of factor VII and a DNA sequence encoding factor IX. DNA sequences encoding derivatives and analogs of factor VII are described in pending U.S. Patent Application Serial Nos. 724,311 and 810,002, as well as published European Patent Application EP 200,421, which are herein incorporated by reference. As the activated form of factor VII has been shown to have therapeutic value, it would be desirable to produce the activated form directly. Direct production would increase yields, reduce the number of production steps and eliminate problems associated with activating blood products.

In another embodiment, a DNA sequence encoding protein C, activated protein C or a modified protein C or activated protein C precursor is inserted into a cell line which has been transfected to express the yeast KEX2 gene. This gene encodes an endopeptidase which cleaves after a dibasic amino acid sequence (Fuller et al., in Leive, ed., Microbiology: 1986, 273-278, 1986). Processing may be further enhanced by also transfecting the cells with the yeast KEX1 gene (Dmochowska et al., Cell 50:573-584, 1987), which encodes an enzyme which removes the basic amino acids from the C-terminus of the protein C light chain. Modified protein C precursors are described in pending commonly assigned U.S. Serial Nos. 924,462 and 130,370, and published European Patent Application EP 266,190, which are incorporated by reference herein in their entirety. Preferred protein C and activated protein C precursors include the amino acid sequence $R_1$-$R_2$-$R_3$-$R_4$-X-$R_5$-$R_6$-$R_7$-$R_8$, wherein $R_1$-$R_8$ are Lys or Arg and X is a peptide bond or a spacer peptide of from one to twelve, preferably from two to eight amino acids, at the cleavage site between the light and heavy chains.

Activated protein C can also be produced in a cell line which has been transfected with DNA sequences encoding an activated protein C precursor, thrombin and thrombomodulin. The thrombin cleaves the activated protein C precursor (two-chain form of protein C) in the presence of thrombomodulin, and activated protein C is secreted by the cells. Activated protein C precursors are described in U.S. Patent Application Serial Nos. 924,462 and 130,370, and in published European Patent Application EP 266,190, which are incorporated herein by reference.

Factor VIIa and Factor IXa can be produced in a cell line transfected to co-express Factor X. The Factor X cleaves the Factor VII or Factor IX, resulting in secretion of the activated clotting factor.

In yet another embodiment, the production of t-PA or a t-PA analog or derivative is enhanced by transfecting a host cell line to produce both t-PA or a t-PA analog or derivative and a protease inhibitor. Suitable protease inhibitors in this regard include TIMP (tissue inhibitor of metalloproteases), trypsin inhibitors and aprotinin, with aprotinin particularly preferred. Analogs and derivatives of t-PA are described in pending U.S. Serial Nos. 822,005 (corresponding to published Japanese Patent Application 63,133,988); 004,995; 053,412; 053,411; 058,061; 058,217; and 125,629 and in European Patent Application Publication Nos. 196,920; 201,153 and 240,334, which are incorporated herein by reference. Expression of t-PA at commercially feasible levels has been difficult because the serine protease activity of t-PA results in detachment of cells from support surfaces. This necessitates the use of agents such as aprotinin in the growth media. The use of aprotinin in the media also allows the production of the single-chain form of t-PA, a desirable therapeutic product. However, aprotinin is both costly and limited in availability.

Plasminogen can be produced in intact form using cells transfected to produce both plasminogen and a protease inhibitor. Variants of plasminogen (as described in U.S. Patent Application Serial No. 053,412) may

EP 0 319 944 B1

also be produced. A particularly preferred protease inhibitor in this regard is alpha-1-antitrypsin, although variants of alpha-1-antitrypsin (U.S. Patent No. 4,711,848) and other protease inhibitors may also be employed. Intracellular plasminogen activation and subsequent degradation have limited the ability to produce recombinant plasminogen at reasonable levels. Inhibition of plasminogen activity by AAT would ameliorate this problem.

Other examples of protein-stabilizer combinations include stabilization of protein C by co-expression of protein S, stabilization of factor VIII by co-expression of von Willebrand factor and stabilization of factor VII by co-expression of tissue factor. BiP (Haas and Wabl, Nature 306:387-389, 1983) may also be used to stabilize various proteins. A cDNA encoding BiP is described by Munro and Pelham (Cell 46:291-300, 1986).

DNA sequences useful in carrying out the present invention may be cloned by standard procedures known in the art. Genomic or cDNA sequences may be used. Many such clones have been described in the literature, including DNAs encoding t-PA (Pennica et al., Nature 301:214-221, 1983), factor VII (Hagen et al., ibid.; Hagen et al., Proc. Natl. Acad. Sci. USA 83:2412-2416, 1986), factor IX (Kurachi and Davie, Proc. Natl. Acad. Sci. USA 79:6461-6464, 1982), plasminogen (Malinowski et al., Biochemistry 23:4243-4250, 1984; Forsgren et al., FEBS Lett. 213:254, 1987), alpha-1-antitrypsin (Long et al., Biochemistry 23:4828-4837, 1984; Kawasaki, U.S. Patent No. 4,559,311), protein C (Foster and Davie, Proc. Natl. Acad. Sci. USA 81:4766-4770, 1984; Foster et al., Proc. Natl. Acad. Sci. USA 82:4673-4677, 1985), prothrombin (Friezner-Degan et al., Biochemistry 22:2087-2097, 1983), factor VIII (Toole et al., Nature 312:342-347, 1984), von Willebrand factor (Lynch et al., Cell 41:4956, 1985; Collins et al., Proc. Natl. Acad. Sci. USA 84:4393-4397, 1987), tissue factor (Spicer et al., Proc. Natl. Acad. Sci. USA 84:5148-5152, 1987) and factor X (Leytus et al., Biochemistry 25:5098-5102, 1986). Additional clones may be obtained by screening cosmid, genomic or cDNA libraries with oligonucleotide probes designed on the basis of amino acid sequence data or with cloned DNA fragments; or through the use of expression libraries which are screened with antibodies to the protein of interest (Young and Davis, Proc. Natl. Acad. Sci. USA 80:1194-1198, 1983), by ligand blotting (Sikela and Hahn, Proc. Natl. Acad. Sci. USA 84:3038-3042, 1987) or by assaying for activity.

The cloned DNA sequences are inserted into suitable expression vectors which are in turn used to transfect or transform suitable eukaryotic host cells.

Expression vectors for use in carrying out the present invention in mammalian cells will comprise a promoter capable of directing the transcription of a cloned gene or cDNA introduced into a mammalian cell. Viral promoters are preferred due to their efficiency in directing transcription. A particularly preferred promoter is the major late promoter from adenovirus 2. Other suitable promoters include the SV40 promoter (Subramani et al., Mol. Cell Biol. 1:854-864, 1981) and the MT-1 (metallothionein gene) promoter (Palmiter et al., Science 222:809-814, 1983). Such expression vectors may also contain a set of RNA splice sites located downstream from the promoter and upstream from the insertion site for the cloned DNA sequence or within the cloned sequence itself. Preferred RNA splice sites may be obtained from adenovirus and/or immunoglobulin genes. Also contained in the expression vectors is a polyadenylation signal, located downstream of the insertion site. Particularly preferred are viral polyadenylation signals, such as the early or late polyadenylation signals from SV40 or the polyadenylation signal from the adenovirus 5 Elb region. Expression vectors useful in carrying out the present invention may also comprise a noncoding viral leader sequence, such as the adenovirus 2 tripartite leader, located between the promoter and the RNA splice sites. Preferred vectors may also include transcriptional enhancer sequences, such as the SV40 enhancer, and translational enhancer sequences, such as the sequences encoding the adenovirus VA RNAs.

Vectors containing cloned DNA sequences may then be introduced into cultured mammalian cells by, for example, calcium phosphate-mediated transfection (Wigler et al., Cell 14:725, 1978; Corsaro and Pearson, Somatic Cell Genetics 7:603, 1981; Graham and Van der Eb, Virology 52:456, 1973) or electroporation Neumann, EMBO J. 1:841-845, 1982). A small fraction of the cells integrate the DNA into the genome or maintain the DNA in non-chromosomal nuclear structures. In order to identify these integrants, a gene that confers a selectable phenotype (a selectable marker) is generally introduced into the cells along with the gene of interest. Preferred selectable markers include genes that confer resistance to drugs, such as neomycin, hygromycin, and methotrexate. If calcium phosphate-mediated transfection is used, selectable markers may be introduced into the cell on a separate plasmid at the same time as the gene of interest, or they may be introduced on the same plasmid. If on the same plasmid, the selectable marker and the gene of interest may be under the control of different promoters or the same promoter, the latter arrangement producing what is known as a dicistronic or polycistronic message. Constructs of this type are known in the art (for example, European Patent Office Publication No. 117,058; U.S. Patent 4,713,339). After the cells have taken up the DNA, they are allowed to grow for a period of time, typically 1-2 days, to begin expressing the gene of interest. Drug selection is then applied to select for the growth of cells which are expressing the selectable marker. When using methotrexate selection, increasing the drug concentration in a stepwise manner allows selection for increased copy number of the cloned sequences, resulting in increased expression levels. Clones of such cells may be screened for production of the protein of interest. Useful screening methods include immunological assays and activity assays.

Preferred mammalian cell lines for use in the present invention include the COS (ATCC CRL 1650), BHK (ATCC CCL 10) and 293 (ATCC 1573) cell lines as well as derivatives and isolates of these cell lines, although other cell lines may be preferred for production of particular proteins. A preferred BHK cell line is the tk⁻ts13 BHK cell line (Waechter and Baserga, Proc. Natl. Acad. Sci. USA 79:1106-1110, 1982), hereinafter referred to as tk⁻BHK cells. Other useful adherent cell lines include Rat Hep I (ATCC CRL 1600), Rat Hep II (ATCC CRL 1548), TCMK (ATCC CCL 139), Human lung (ATCC CCL 75.1), Human hepatoma (ATCC HTB-52), Hep G2 (ATCC HB 8065), Mouse liver (ATCC CC 29.1) and DUKX cells (Urlaub and Chasin, Proc. Natl. Acad. Sci. USA 77:4216-4220, 1980). Useful suspension cell lines include AtT-20 (ATCC CCL 89), MOLT-4 (ATCC CRL 1582), BW5147.G.1.4.OUA$^R$.1 (ATCC CRL 1588), S194/5.XXO.BU.I (ATCC TIB 20), EL4.BU.1.OUA$^r$.1.1 (ATCC TIB 41), Sp 2/0-Ag14 (ATCC CRL 1581), J558L (Oi et al., Proc. Natl. Acad. Sci. USA 80:825-829, 1983) and Raji (ATCC CCL 86).

In general, a host cell line will be selected on the basis of its ability to produce the protein of interest at a high level or its ability to carry out at least some of the processing steps necessary for the biological activity of the protein. In this way, the number of cloned DNA sequences which must be transfected into the host cell line may be minimized and overall yield of biologically active protein may be maximized. However, the present invention allows one to produce virtually any protein in practically any cell line which can be cultured in vitro.

DNA sequences encoding the protein of interest and the processing and/or stabilizing protein(s) may be introduced into the cell on the same vector or on different vectors. It is preferred to use a single vector with one selectable marker in order to minimize problems which can result from marker instability. Genes or cDNAs on a vector may be controlled by separate promoters or by a single promoter. In the latter arrangement, which gives rise to a polycistronic message, the genes or cDNAs will be separated by translational stop and start signals. When transfecting with a large number of DNA sequences, practical limitations on vector size may necessitate the use of two or more vectors, each with its own selectable marker. Two or more vectors may, of course, be used whenever co-expression of a protein of interest and a stabilizing or processing protein is desired.

Other eukaryotic cells, including yeast and filamentous fungi, may also be used within the present invention. These lower eukaryotic hosts provide certain advantages over mammalian cell lines, including ease and economy of culturing and existing industrial fermentation capacity. By manipulating cells as described herein, fungal cells and other eukaryotic cells capable of expressing cloned DNA sequences can be used to produce virtually any protein of interest.

For example, cells of bakers' yeast (Saccharomyces cerevisiae) can be transformed with cloned foreign DNA sequences and cultured to high cell densities, and will express the cloned DNA and secrete the foreign proteins. In some instances, however, the foreign proteins are not secreted or are inactive due to a lack of proper processing or proteolytic degradation. Yeast cells cannot, for instance, gamma-carboxylate proteins or add mammalian-type complex carbohydrate chains to glycoproteins. According to the present invention, this lack of processing can be overcome by transforming yeast host cells with DNA sequences encoding the missing protein(s) (e.g., gamma-carboxylase, glycosylating enzymes). Degradation of foreign proteins may be overcome by transforming the cells to produce protease inhibitors or proteins which bind to the protein of interest. An example of a suitable binding protein is BiP. Binding proteins may also enhance secretion of a foreign protein by altering its conformation. In addition, yeast cells can be transformed to produce foreign proteases, enabling them to produce and secrete active forms of foreign proteins (e.g., mammalian serine proteases) which require specific cleavage for secretion and/or biological activity. Junction points between secretory peptides and mature proteins may be engineered so that cleavage by a co-expressed protease (e.g., thrombin) releases the mature protein.

Eukaryotic microorganisms, such as the yeast Saccharomyces cerevisiae, or filamentous fungi including Aspergillus species, may be transformed according to known procedures. Aspergillus species may be transformed, for example, according to the method of Yelton et al. (Proc. Natl. Acad. Sci. USA 81:1740-1747, 1984). Particularly preferred species of Aspergillus include A. nidulans, A. niger, A. oryzae, and A. terreus. Techniques for transforming yeast are described, for example, by Beggs (Nature 275:104-108, 1978). Expression vectors for use in yeast include YRp7 (Struhl et al., Proc. Natl. Acad. Sci. USA 76:1035-1039, 1979), YEp13 (Broach et al., Gene 8:121-133, 1979), pJDB248 and pJDB219 (Beggs ibid.), and derivatives thereof. Such vectors will generally comprise a selectable marker, such as the nutritional marker TRP1, which allows selection in a host strain carrying a trpl mutation, or the POT1 selectable marker, which permits selection in a tpi-strain grown in rich medium (Kawasaki and Bell, EP 171,142). Preferred promoters for use in yeast expression vectors include promoters from yeast glycolytic genes (Hitzeman et al., J. Biol. Chem. 255:12073-12080, 1980; Alber and Kawasaki, J. Mol. Appl. Genet. 1:419-434, 1982; Kawasaki, U.S. Patent No. 4,599,311) or alcohol dehydrogenase genes (Young et al., in Genetic Engineering of Microorganisms for Chemicals, Hollaender et al., eds., p. 335, Plenum, New York, 1982; and Ammerer,, Meth. in Enzymology 101:192-201, 1983). To facilitate secretion of a protein of interest produced in a yeast transformant and to obtain proper processing, a signal sequence will generally be provided. Preferably the signal sequence will be obtained from a yeast gene encoding a secreted protein. A particularly preferred signal sequence is the pre-pro region of the MFαl gene (Kurjan and Herskowitz, Cell 30:933-943, 1982; Kurjan et al., U.S. Patent 4,546,082 and Singh, EP 123,544).

Co-expression of DNA sequences in yeast may be achieved in several ways. It is preferred that the expression unit for the processing or stabilizing protein be integrated into the host cell genome and an isolate which stably produces the processing or stabilizing activity be selected. An expression vector containing the DNA sequence for the protein of interest is then transformed into the host strain. Alternatively, the two expression units may be on different autonomously replicating expression vectors with different selectable markers or on a single expression vector.

In a preferred embodiment, a DNA sequence encoding a foreign processing protein is inserted into a DNA sequence encoding a yeast protein having a similar function. The insertion is designed to substitute the foreign sequence for yeast sequences encoding the processing function(s) of the yeast protein. A particularly preferred such yeast protein is the KEX2 gene product. This protein has been analyzed and its catalytic and other domains have been characterized (Fuller et al., Yeast Cell Biology. Cold Spring Harbor Laboratory, Cold Spring Harbor, New York, 1987, page 181). The resulting hybrid sequence, comprising the foreign DNA sequence and yeast sequences encoding transport and cellular localization domains, is then joined to a yeast promoter and terminator. Preferably, the promoter is a strong promoter, such as the TPI1 promoter. This construct may also contain flanking sequences to target the expression unit to a particular integration site in the host cell genome.

Proteins produced according to the present invention may be purified from cell-conditioned media by a variety of procedures known in the art, which may be selected according to the physicochemical characteristics of the particular protein. Suitable methods include affinity chromatography, ion exchange chromatography, gel filtration, high performance liquid chromatography, and combinations of these methods.

Proteins produced according to the present invention may be used within compositions for pharmaceutical, industrial, research and other purposes. For pharmaceutical use, the proteins will generally be combined with a physiologically acceptable carrier or diluent, such as sterile water or sterile saline, and packaged in individual doses in sterile vials. Alternatively, the proteins may be packaged in lyophilized form and reconstituted prior to administration. Administration will generally be by injection or infusion. Pharmaceutical compositions may further contain additional proteins or other compounds of therapeutic value, adjuvants, local anesthetics, etc.

The following examples are offered by way of illustration and not by way of limitation.

EXAMPLES

Except where noted, conventional molecular biology techniques were employed. Restriction endonucleases and other DNA modification enzymes (e.g., T4 polynucleotide kinase, calf alkaline phosphatase, DNA polymerase I Klenow fragment, T4 polynucleotide ligase) were obtained from Bethesda Research Laboratories, New England Biolabs or Boehringer-Mannheim and used according to the suppliers' instructions or as described in Maniatis et al., eds., Molecular Cloning: A Laboratory Manual, Cold Spring Harbor Laboratory, Cold Spring Harbor, NY, 1982. Oligonucleotides were synthesized on an Applied Biosystems model 380A DNA synthesizer and purified by polyacrylamide gel electrophoresis on denaturing gels.

Example 1

Production of Tissue Plasminogen Activator

Tissue plasminogen activator (t-PA) cDNAs have been described in the literature. See, for example, Pennica et al. (ibid.), Kaufman et al. (Mol. Cell. Biol. 5:1750-1759, 1985) and Verheijen et al. (EMBO J. 5:3525-3530, 1986). A cDNA clone comprising the coding sequence for mature t-PA was constructed in the inventors' laboratory using as starting material mRNA from the Bowes melanoma cell line (Rijken and Collen, J. Biol. Chem. 256:7035-7041, 1981). This cDNA was then used to construct the plasmid pDR1296. E. coli strain JM83 transformed with pDR1296 has been deposited with American Type Culture Collection under accession number 53347.

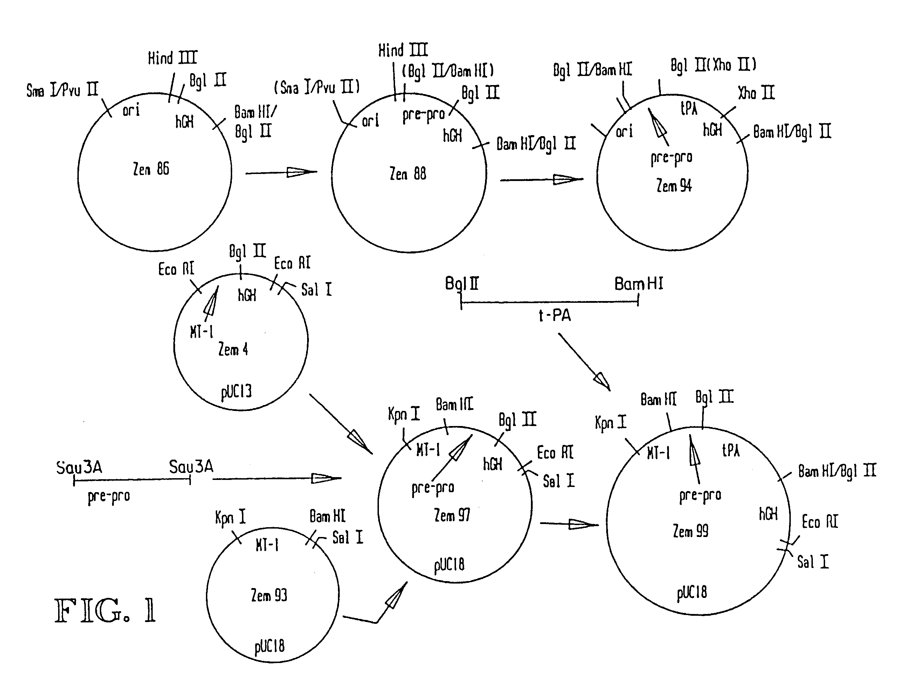

The cDNA in pDR1296 was extended to include the coding sequence for the pre-pro peptide of t-PA by joining it to a fragment constructed from synthesized oligonucleotides. The synthesized pre-pro fragment was inserted into Bam HI-digested pUC8. Plasmid pIC19R (Marsh et al., Gene 32:481-486, 1984) was digested with Sma I and Hind III. The ori region of SV40 from map position 270 (Pvu II) to position 5171 (Hind III) was then ligated to the linearized pIC19R to produce plasmid Zem67. This plasmid was then cleaved with Bgl II and the terminator region from the human growth hormone gene (De Noto et al., Nuc. Acids Res. 9:3719-3730, 1981) was inserted as a Bgl II-Bam HI fragment to produce plasmid Zem86 (Figure 1). The synthesized t-PA pre-pro sequence was removed from the pUC8 vector by digestion with Bam HI and Xho II. This fragment was inserted into Bgl II-digested Zem86 to produce plasmid Zem88. Plasmid pDR1296 was digested with Bgl II and Bam HI and the t-PA cDNA fragment was isolated and inserted into Bgl II-cut Zem88. The resultant plasmid was designated Zem94.

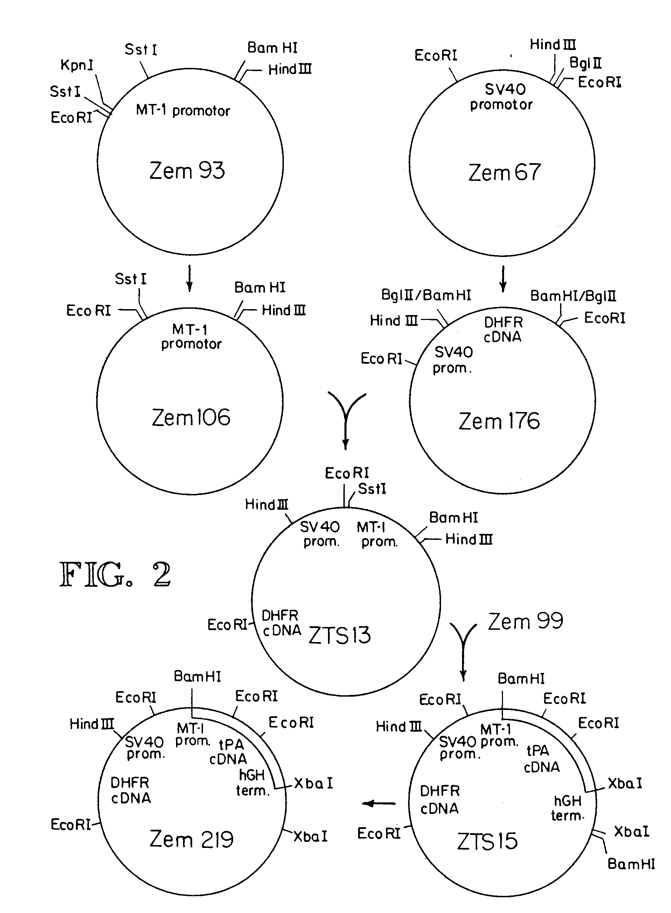

The vector Zem99, comprising the MT-1 promoter, complete t-PA coding sequence, and the human growth hormone (hGH) terminator, was then assembled as shown in Figure 1. A Kpn I-Bam HI fragment comprising the MT-1 promoter was isolated from MThGH111 (Palmiter et al., Science 222:809-814, 1983) and inserted into pUC18 to construct Zem93. Plasmid EV142, comprising MT-1 and hGH sequences in the pBR322 derivative pBX322 (Palmiter et al., ibid.), was digested with Eco RI and the fragment comprising the MT-1 promoter and hGH terminator sequences was isolated. This fragment was cloned into Eco RI-digested pUC13 to construct plasmid Zem4. Zem93 was then linearized by digestion with Bam HI and Sal I. Zem4 was digested with Bgl II and Sal I and the hGH terminator was purified. The t-PA pre-pro sequence was removed from the pUC9 vector as a Sau 3A fragment. The three DNA fragments were then joined and a plasmid having the structure of Zem97 (Figure 2) was selected. Zem97 was cut with Bgl II and the Bgl II-Bam HI t-PA fragment from pDR1296 was inserted. The resultant vector was designated Zem99.

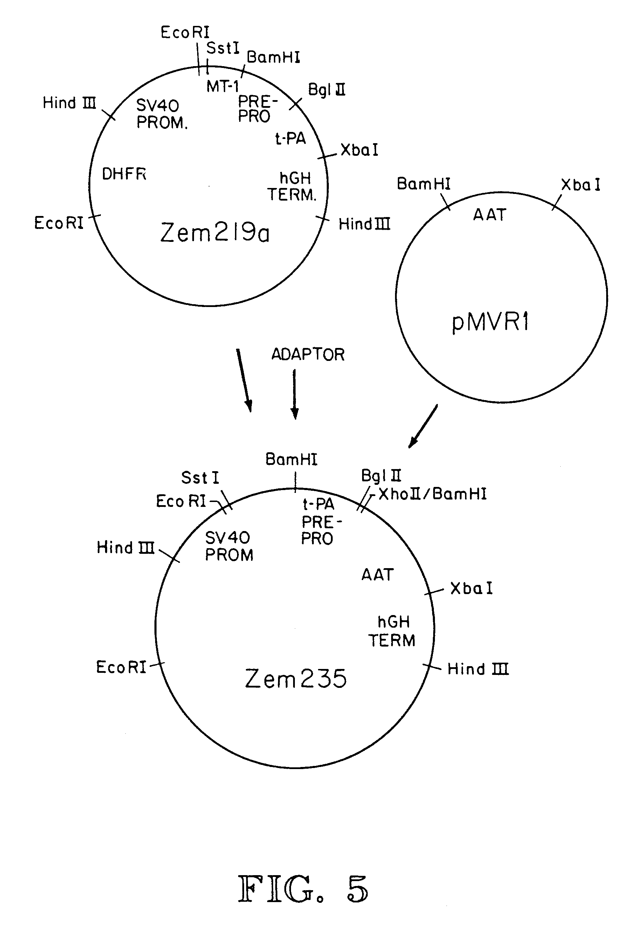

Plasmid pSV2-DHFR (Subramani et al., ibid) was digested with Cfo I, and the fragment containing the DHFR cDNA and the 3′ attached SV40 sequences was isolated, repaired, and ligated to Bam HI linkers. After digestion with Bam HI, an approximately 800 bp fragment containing the entire cDNA and the SV40 terminator region was purified and ligated to Bam HI-digested pUC8. As shown in Figure 2, Zem67 was digested with Bgl II and ligated with the Bam HI DHFR-SV40 fragment to generate plasmid Zem176. Plasmid Zem93 was digested with Sst I and re-ligated to generate plasmid Zem106, in which approximately 600 bp of sequence 5′ to the MT-1 promoter was eliminated. Plasmid Zem106 was digested with Eco RI and ligated to the Eco RI fragment containing the DHFR gene from plasmid Zem176. The resulting plasmid was designated Zts13. Plasmid Zts13 was digested with Bam HI and ligated to the Bam HI fragment from plasmid Zem99 containing the entire t-PA coding region and hGH terminator sequence. The resulting plasmid was designated Zts15. Zts15 was partially digested with Bam HI, repaired and re-ligated to generate plasmid Zem219, in which the 3′ Bam HI site was destroyed (Figure 2). Zem219 was then partially digested with Xba I, repaired and religated to generate plasmid Zem219a, in which the 3′ Xba I site was destroyed (Figure 5).

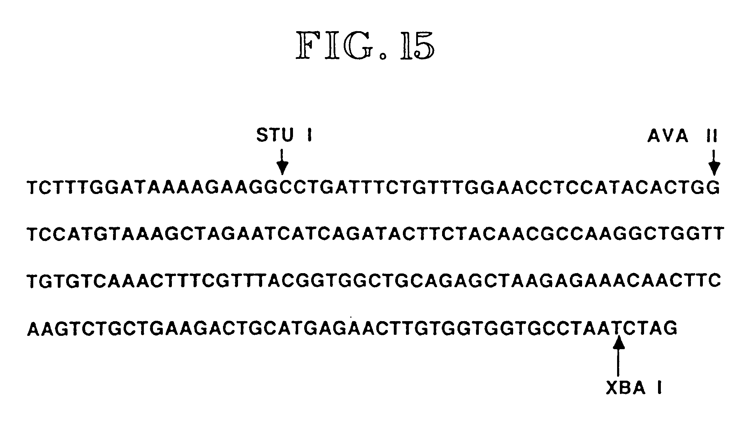

A DNA sequence encoding aprotinin was then inserted into Zem219a in place of the sequence encoding mature t-PA. Zem219a was digested with Bgl II and Xba I, and the vector fragment was recovered. Plasmid pKFN-414, a pUC-based plasmid containing a synthetic aprotinin gene, was digested with Ava II and Xba I, and the ~150 bp aprotinin fragment was recovered. (The sequence of the synthetic aprotinin gene is shown in Figure 15.) This fragment lacks the 5′ end of the aprotinin coding sequence. Oligonucleotides ZC1908 (sense strand, 5′ GAT CTA GGC CTG ATT TCT GTT TGG AAC CTC CAT ACA CTG 3′) and ZC1909 (anti-sense, 5′ GAC CAG TGT ATG GAG GTT CCA AAC AGA AAT CAG GCC TA 3′) were annealed to provide the 5′ end of the aprotinin sequence and a Bgl II site to join the aprotinin sequence in frame with the t-PA pre-pro sequence. The ~150 bp aprotinin fragment, the annealed oligonucleotides and the Zem219a fragment were then joined to construct Zem252.

Plasmids Zem219a and Zem252 were cotransfected into tk⁻BHK cells by the calcium phosphate procedure. Transfected cells were selected with methotrexate and screened for production of t-PA. Positive clones were radiolabelled and culture media were screened for aprotinin production using a rabbit anti-aprotinin antibody. Aprotinin-producing clones were selected.

Example 2

Production of Plasminogen

A. Cloning of Alpha-1-Antitrypsin cDNA

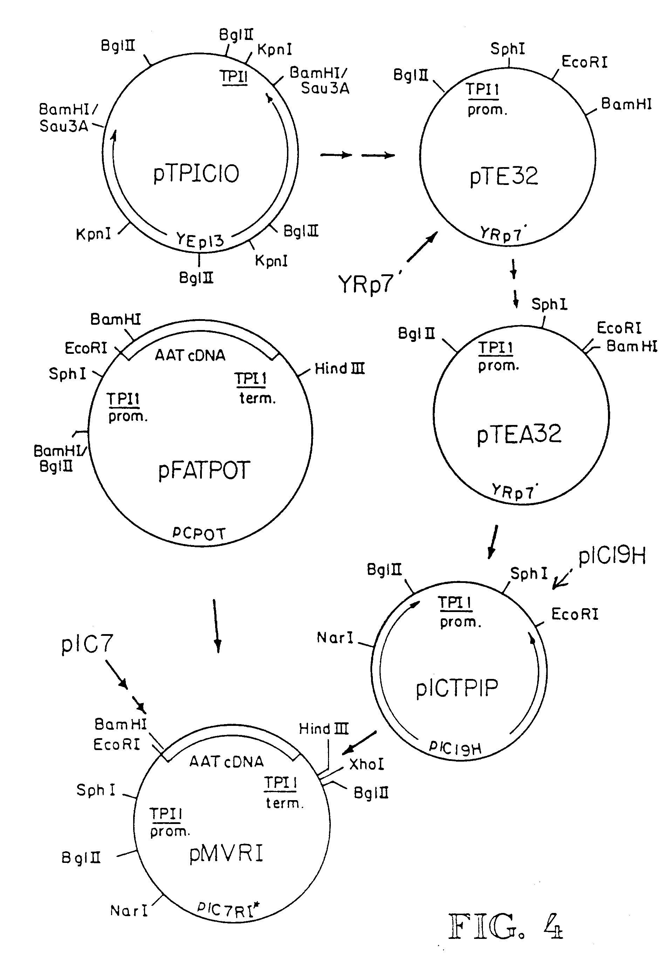

A cDNA coding for the predominant form of human α-1-antitrypsin (AAT) was isolated from a human liver cDNA library by conventional procedures using the baboon sequence (Kurachi et al., Proc. Natl. Acad. Sci. USA 78:6826-6830, 1980; and Chandra et al., Biochem. Biophys. Res. Comm. 103:751-758, 1981) as a DNA hybridization probe. The library was constructed by inserting human liver cDNA into the Pst I site of the plasmid pBR322 (Bolivar et al., Gene 2:95-113, 1977). The AAT cDNA was isolated from the library as a 1500 base pair (bp) Pst I fragment. This fragment was inserted into the Pst I site of pUC13 to produce the plasmid pUCαl. In pUCαl the AAT sequence is flanked on the 3′ end by Xba I and Eco RI sites in the polylinker. This cDNA sequence was used to construct the plasmid PFATPOT, illustrated in Figure 4. Plasmid PFATPOT has been deposited with ATCC as a Saccharomyces cerevisiae strain E18 transformant, accession number 20699.

The AAT cDNA was then joined to the TPI1 (triose phosphate isomerase gene) terminator in the plasmid pMVR1. This plasmid further comprises the TPI1 promoter and was assembled in the following manner. Plasmid pIC7 (Marsh et al., Gene 32:481-486, 1984) was digested with Eco RI, the fragment ends were blunted with DNA polymerase I (Klenow fragment), and the linear DNA was recircularized using T4 DNA ligase. The resulting plasmid was used to transform E. coli RR1. Plasmid DNA was prepared from the transformants and screened for the loss of the Eco RI site. A plasmid having the correct restriction pattern was designated pIC7RI*. The TPI1 promoter fragment was obtained from plasmid pTPIC10 (Alber and Kawasaki, J. Molec. Appl. Genet. 1:419-434, 1982) as illustrated in Figure 4. This plasmid was cut at the unique Kpn I site within the TPI1 gene, the TPI1 coding region was removed with Bal31 exonuclease, and an Eco RI linker (sequence: GGAATTCC) was added to the 3′ end of the promoter. Digestion with Bgl II and Eco RI yielded a TPI1 promoter fragment having Bgl II and Eco RI sticky ends. This fragment was then joined to plasmid YRp7′ (Stinchcomb et al., Nature 282:3943, 1979) which had been cut with Bgl II and Eco RI. The resulting plasmid, TE32, was cleaved with Eco RI and Bam HI to remove a portion of the tetracycline resistance gene. The linearized plasmid was then recircularized by the addition of the previously described Eco RI-Bam HI linker to produce plasmid TEA32. Plasmid TEA32 was digested with Bgl II and Eco RI and the approximately 900 bp partial TPI1 promoter fragment was gel purified. Plasmid pIC19H (Marsh et al., ibid.) was cut with Bgl II and Eco RI and the vector fragment was gel purified. The TPI1 promoter fragment was then ligated to the linearized pIC19H and the mixture was used to transform E. coli RR1. Plasmid DNA was prepared and screened for the presence of an approximately 900 bp Bgl II-Eco RI fragment. A correct plasmid was selected and designated pICTPIP. Plasmid pIC7RI* was digested with Hind III and Nar I and the 2500 bp fragment was gel purified. The partial TPI1 promoter fragment (ca. 900 bp) was removed from pICTPIP using Nar I and Sph I and was gel purified. pFATPOT was digested with Sph I and Hind III and the 1750 bp fragment comprising a portion of the TPI1 promoter, the AAT cDNA, and the TPI1 terminator was gel purified. The pIC7RI* fragment, the TPI1 promoter fragment, and the TPI1 promoter-AAT-TPI1 terminator fragment from pFATPOT were then combined in a triple ligation to produce pMVR1 (Figure 4).

B. Construction of Alpha-1-Antitrypsin Expression Vector and Transfection of BHK Cells

For expression and secretion of alpha-1-antitrypsin by transfected mammalian cells, the Bam HI to Xba I fragment from plasmid pMVR1, containing the entire coding region for AAT from amino acid number 2, was isolated and inserted into Bam HI, Xba I digested Zem219a (Example 1).

To construct the AAT expression vector, Zem219a was digested with Bgl II and Xba I and the t-PA coding sequence was removed. Oligonucleotides ZC1173 (5′GAT CTT CA3′) and ZC1174 (5′GAT CTG AA3′) were annealed to form a Bgl II-Xho II adaptor. The AAT fragment, adaptor and linearized vector were then joined in a three-part ligation to construct Zem235 (Figure 5). The adaptor correctly aligned the reading frames of the t-PA pre-pro and AAT sequences and restored the glu codon which was missing from the 5′ end of the AAT sequence.

Plasmid Zem235 was transfected into tk⁻ BHK cells by electroporation. Methotrexate selection was applied after 48 hours, and after two weeks a number of clones were picked, expanded and characterized for levels of alpha-1-antitrypsin secreted into the culture media. One clone, which secreted AAT at a rate of about 20 μg/ml/day, was selected and designated 235-6.

C. Construction of Plasminogen Expression Vector and Transfection of Cells

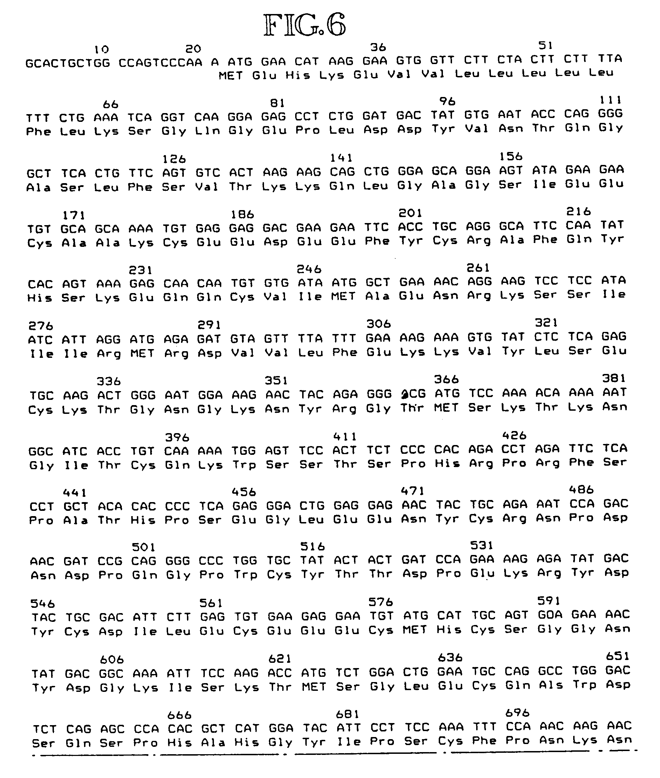

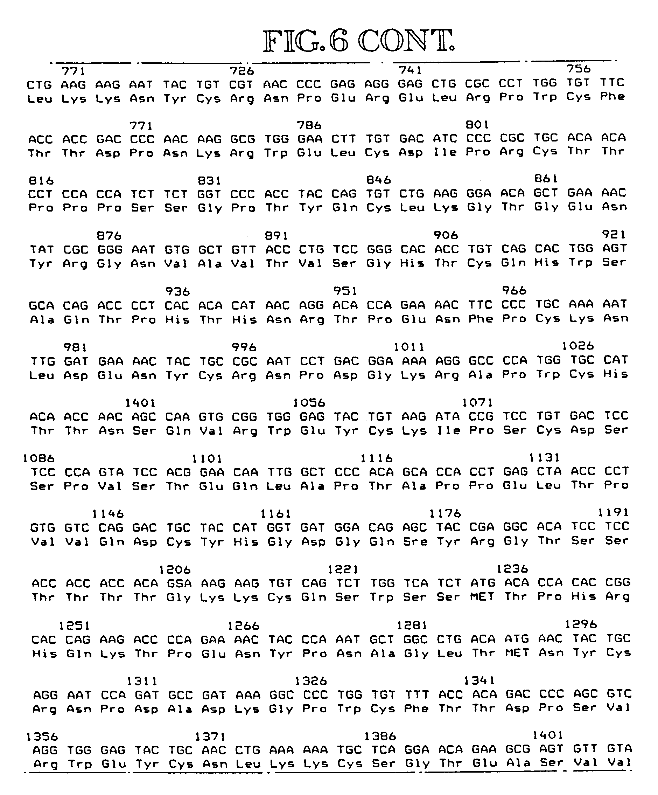

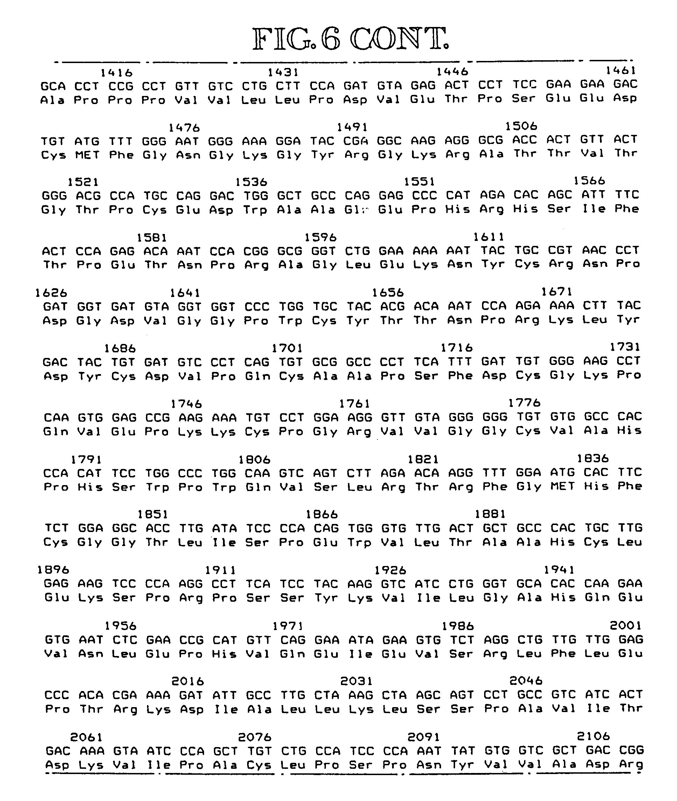

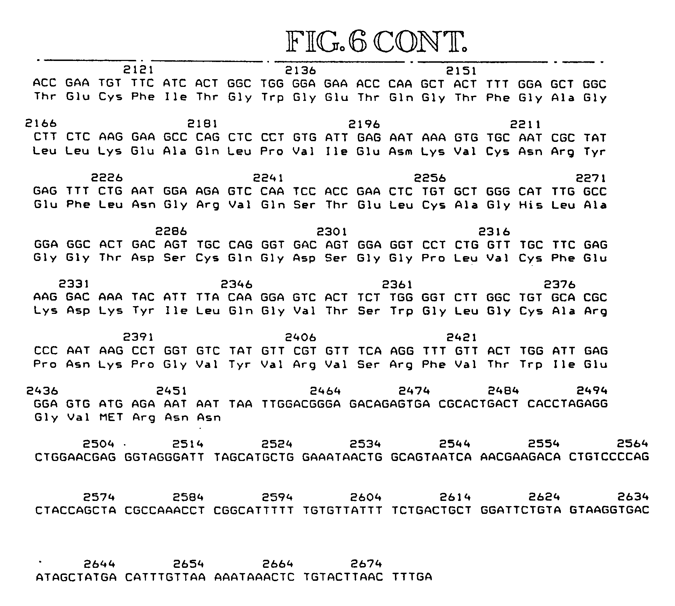

A cDNA encoding plasminogen was obtained from Dr. Mark Markson of the University of Washington, Seattle, Washington. The clone had been isolated from a lambda phage library screened with a partial cDNA clone described by Malinowski et al. (ibid.). The sequence of the cDNA is shown in Figure 6.

Lambda phage DNA was prepared from the positive clone according to conventional procedures. Phage DNA was subjected to a partial Eco RI digestion and an approximately 2800 bp Eco RI fragment containing the entire coding region was recovered and cloned into the Eco RI site of pUC19 to construct plasmid pUC19-Plg.

The 183 bp Bal I-Eco RI fragment containing the 5′ end of the coding sequence was isolated from pUC19-Plg and cloned into Sma I, Eco RI-digested pUC18 in order to add a Bam HI site to the 5′ end. The resultant plasmid was digested with Bam HI and Eco RI and the 190 bp fragment was isolated.

Plasmid pUC19-Plg was digested with Eco RI and Eco RV and the fragment containing the middle region of the cDNA was isolated.

To obtain the 3′ portion of the plasminogen cDNA, a fragment beginning at the codon for amino acid 541 was subcloned in pUC118. The resulting plasmid was digested with Eco RV and Xba I and the approximately 660 bp 3′ plasminogen fragment was isolated.

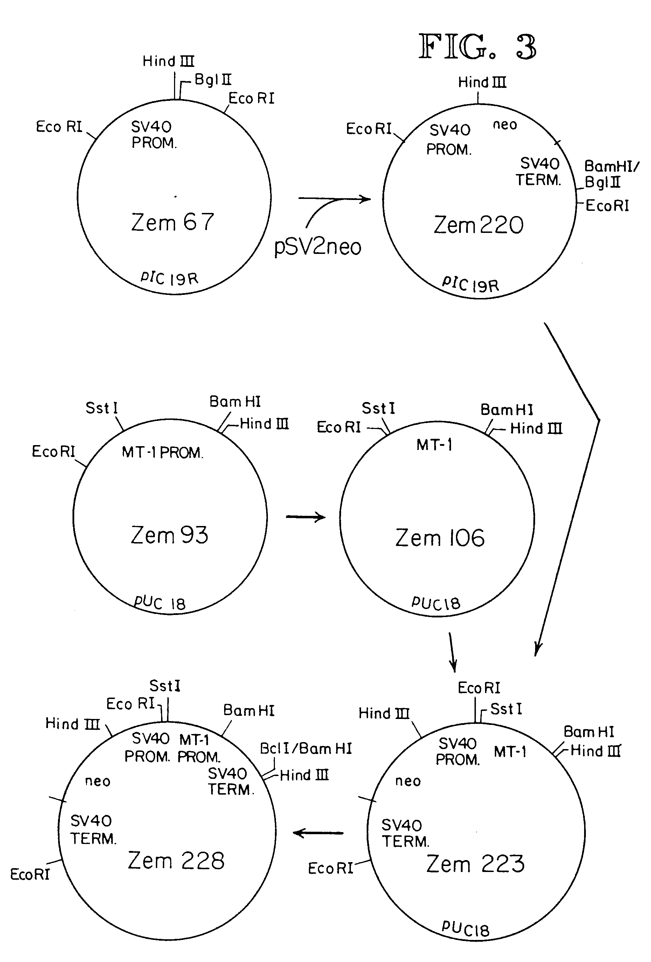

The three plasminogen cDNA fragments (Bam HI-Eco RI, Eco RI-Eco RV and Eco RV-Xba I) were combined with Bam HI, Xba I digested Zem219b for ligation to generate plasmid 219b-Plg. (Zem219b was derived from Zem219a by digesting that vector with Bam HI and Xba I, removing the t-PA cDNA sequences, and ligating the vector fragment with a Bam HI-Xba I adaptor.) The Bam HI plasminogen fragment was then removed from Zem219b-Plg and inserted into Bam HI-digested Zem228. To construct Zem228, Zem67 was digested with Hind III and Bgl II and the Hind III-Bam HI neo+SV40 terminator fragment from pSV2neo (Southern and Berg, J. Mol. Appl. Genet. 1:327-341, 1982) was inserted. The resultant vector was designated Zem220. Plasmid Zem93 was digested with Sst I to remove upstream MT-1 sequences and the vector was recircularized to construct Zem106. Zem220 was digested with Eco RI and the fragment containing the expression unit of SV40 promoter-neo-SV40 terminator was recovered and joined to Eco RI-digested Zem106. The resultant vector, designated Zem223, contained the SV40 and MT-1 promoters in opposite orientation. Zem223 was digested with Bam HI and a 237 bp Bcl I-Bam HI SV40 terminator fragment was inserted. The resultant plasmid was designated Zem228 (Figure 3). The Zem228-derived plasminogen expression vector was designated Zem228-Plg.

Zem228-Plg was transfected into the ATT-expressing cell line 235-6 by the electroporation procedure, essentially as described by Neumann (ibid.). Colonies were selected and assayed for plasminogen production by enzyme-linked immunosorbent assay (ELISA). The assay used a goat polyclonal antiserum to human plasminogen (American Diagnostica) as the capture antibody. Immunoreactive material was detected by means of biotinylated rabbit polyclonal antibody against human plasminogen and avidin-conjugated horseradish peroxidase.

Ten positive clones were put into six-well tissue culture dishes. When the cells were 80-90% confluent, they were put into cysteine-deficient medium for one hour. Fifty microcuries of $^{35}$S-cysteine was added to each well (1 ml culture volume/well), the cells were incubated overnight, and the media were harvested for assay.

The cells were washed with cold PBS, and an extract was prepared to assay for cytoplasmic plasminogen. The cells were suspended in 1 ml RIP A buffer (10 mM Tris pH 7.4, 1% deoxycholate, 1% Triton X-100, 0.1% SDS, 5 mM EDTA, 0.7 M NaCl). The lysates were freeze-thawed two times on dry ice and centrifuged in the cold for 15 minutes at 10,000 rpm. The supernatants were then used for radioimmune precipitation.

Media and cell extract samples were assayed for plasminogen by radioimmune precipitation. Samples (in a volume of 0.1 to 1.0 ml) were combined with 5-10 $\mu$l of preimmune serum, incubated on ice for 1 hour and then mixed with 50-100 $\mu$l of Staphylococcus aureus (Pansorbin, Sigma Chemical Co., St. Louis, MO) and incubated for 1 hour on ice. After centrifugation, the supernatants were mixed with 5 $\mu$l of rabbit polyclonal antiserum to human plasminogen (Boehringer-Mannheim) and incubated on ice for one hour. Fifty $\mu$l of Staphylococcus aureus was added and the mixture was incubated one hour on ice. The cells were pelleted and the pellets were washed with one ml PBS containing 0.5% NP-40, 0.1% SDS. The washed cells were pelleted, resuspended in 50 $\mu$l PBS plus 50 $\mu$l 2X loading buffer (0.1 M Tris pH 6.8, 16% glycerol, 3.2% SDS, 8% $\beta$-mercaptoethanol, 0.002% bromphenol blue), boiled 5 minutes and electrophoresed on a 10% polyacrylamide gel. Proteins were visualized by silver staining and autoradiography.

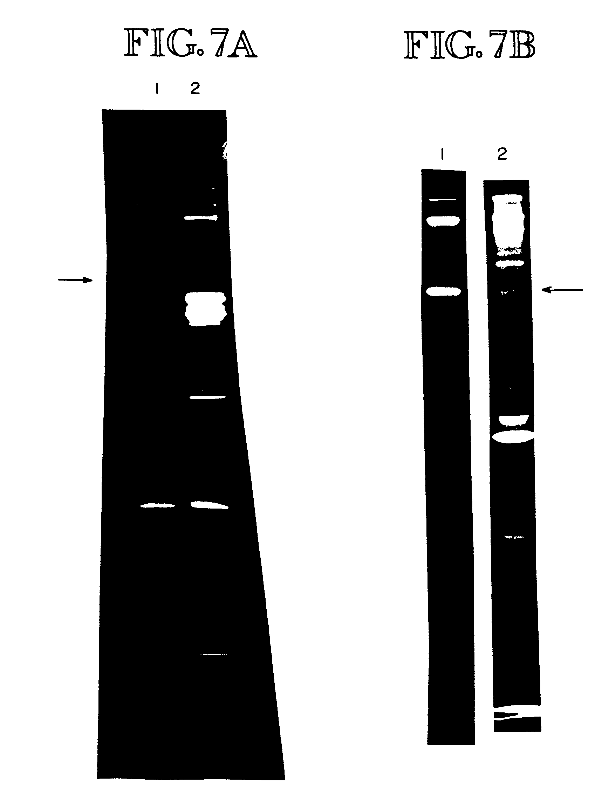

Assay results showed that AAT-producing cells secreted full-length plasminogen into the culture media. In contrast, control BHK cells (i.e., cells transfected with 219b-Plg but not Zem235) did not secrete detectable amounts of plasminogen and contained degraded plasminogen in the cytoplasm (Figure 7).

Example 3

Production of Protein C

A. Protein C cDNA Cloning

A cDNA coding for a portion of human protein C was prepared as described by Foster and Davie (ibid.). Briefly, a λgt11 cDNA library was prepared from human liver mRNA by conventional methods. Clones were screened using $^{125}$I-labeled affinity-purified antibody to human protein C, and phage were prepared from positive clones by the plate lysate method (Maniatis et al., ibid.), followed by banding on a cesium chloride gradient. The cDNA inserts were removed using Eco RI and subcloned into plasmid pUC9 (Vieira and Messing, Gene 19:259-268, 1982). Restriction fragments were subcloned in the phage vectors M13mp10 and M13mp11 (Messing, Meth. in Enzymology 101:20-77, 1983) and sequenced by the dideoxy method (Sanger et al., Proc. Natl. Acad. Sci. USA 74:5463-5467, 1977). A clone was selected which contained DNA corresponding to the known partial sequence of human protein C (Kisiel, J. Clin. Invest. 64:761-769, 1979) and encoded protein C beginning at amino acid 64 of the light chain and extending through the heavy chain and into the 3′ non-coding region. This clone was designated λHC1375. A second cDNA clone coding for protein C from amino acid 24 was identified. The insert from this clone was subcloned into pUC9 and the plasmid designated pHCλ6L. This clone encodes a major portion of protein C, including the heavy chain coding region, termination codon, and 3′ non-coding region.

The cDNA insert from λHC1375 was nick translated using $\alpha$-$^{32}$P dNTP's and used to probe a human genomic library in phage λCharon 4A (Maniatis et al., Cell 15:687-702, 1978) using the plaque hybridization procedure of Benton and Davis (Science 196:181-182, 1977) as modified by Woo (Meth. in Enzymology 68:381-395, 1979). Positive clones were isolated and plaque-purified (Foster et al., Proc. Natl. Acad. Sci. USA 82:4673-4677, 1985, herein incorporated by reference). Phage DNA prepared from positive clones (Silhavy et al., in Experiments with Gene Fusion, Cold Spring Harbor Laboratory, 1984) was digested with Eco RI or Bgl II and the genomic inserts were purified and subcloned in pUC9. Insert restriction fragments were subcloned into M13 vectors and sequenced to confirm their identity and establish the DNA sequence of the entire gene.

A genomic fragment containing an exon corresponding to amino acids -42 to -19 of the pre-pro peptide of protein C was isolated, nick translated, and used as a probe to screen a cDNA library constructed by the technique of Gubler and Hoffman (Gene 25:263-269, 1983) using mRNA from Hep G2 cells. This cell line was derived from human hepatocytes and was previously shown to synthesize protein C (Fair and Bahnak, Blood 64:194-204, 1984). Ten positive clones comprising cDNA inserted into the Eco RI site of phage λgt11 were isolated and screened with an oligonucleotide probe corresponding to the 5′ non-coding region of the protein C gene. One clone was also positive with this probe and its entire nucleotide sequence was determined. The cDNA contained 70 bp of 5′ untranslated sequence, the entire coding sequence for human prepro-protein C, and the entire 3′ non-coding region corresponding to the second polyadenylation site.

B. Construction of Vector pD5

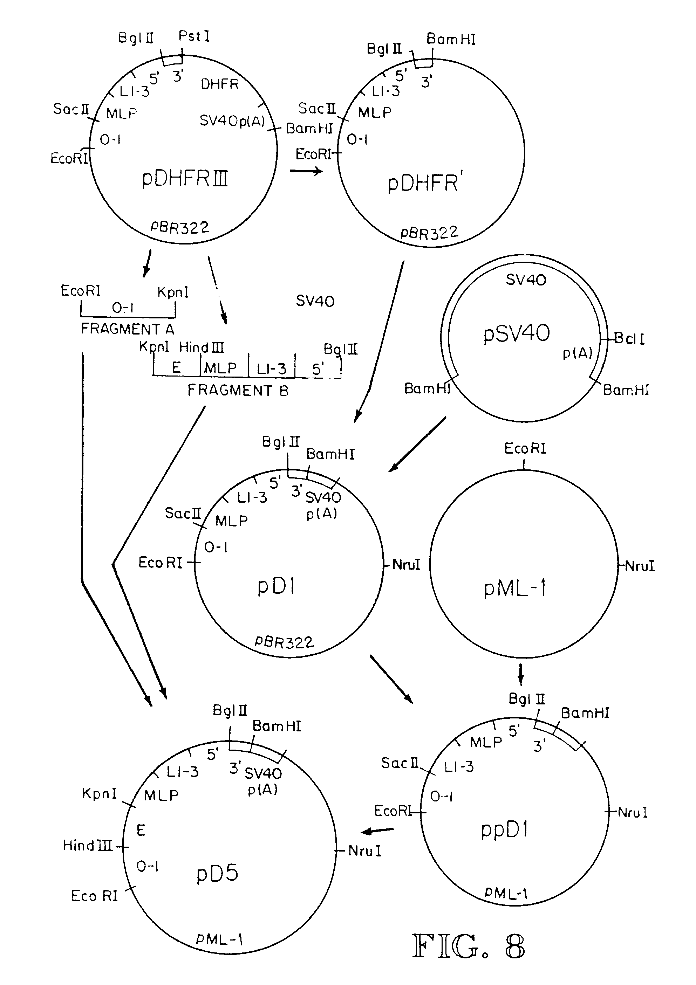

The vector pD5 was derived from pDHFRIII as shown in Figure 8. The Pst I site immediately upstream from the DHFR sequence in pDHFRIII (Berkner and Sharp, Nuc. Acids Res 13:841-857, 1985) was converted to a Bam HI site by digesting 10 μg of plasmid with 5 units of Pst I for 10 minutes at 37°C in 100 μl buffer A (10 mM Tris pH 8, 10 mM MgCl2, 6 mM NaCl, 7 mM $\beta$-MSH). The DNA was phenol extracted, EtOH precipitated, and resuspended in 40 μl buffer B (50 mM Tris pH 8, 7 mM MgCl2, 7 mM $\beta$-MSH) containing 10 mM dCTP and 16 units T4 DNA polymerase and incubated at 12°C for 60 minutes. Following EtOH precipitation, the DNA was ligated to 2.5 μg kinased Bam HI linkers in 14 μl buffer C (10 mM Tris pH 8, 10 mM MgCl2, 1 mM DTT, 1.4 mM ATP) containing 400 units T4 polynucleotide ligase for 12 hours at 12°C. Following phenol extraction and EtOH precipitation, the DNA was resuspended in 120 μl buffer D (75 mM KCl, 6 mM Tris pH 7.5, 10 mM MgCl2, 1 mM DTT), digested with 100 units Bam HI for 60 minutes at 50°C, then electrophoresed through agarose. The 4.9 kb DNA fragment containing pBR322 and vector sequences (10 μg) was isolated from the gel, ligated in 10 μl buffer C containing 50 units T4 polynucleotide ligase for 2 hours at 12°C, and used to transform E. coli HB101. Positive colonies were identified by rapid DNA preparation analysis, and plasmid DNA (designated pDHFR′) was prepared.

Plasmid pD1 was then generated by first cleaving pSV40 (comprising Bam HI digested SV40 DNA cloned into the Bam HI site of PML-1 [Lusky and Botchan, Nature 293:79-81, 1981]) (25 μg) in 100 μl buffer D with 25 units Bcl I for 60 minutes at 50°C, followed by the addition of 50 units of Bam HI and additional incubation at 37°C for 60 minutes. Plasmid pDHFR′ was linearized with Bam HI and treated with calf intestinal phosphatase. DNA fragments were resolved by agarose gel electrophoresis, and the 4.9 kb pDHFR′ fragment and 0.2 kb SV40 fragment were isolated. These fragments (200 ng pDHFR′ DNA and 100 ng SV40 DNA) were incubated in 10 μl buffer C containing 100 units T4 polynucleotide ligase for 4 hours at 12°C, and the resulting construct (pD1) was used to transform E. coli RR1.

As shown in Figure 8, plasmid pD1 was modified by deleting the "poison" sequences in the pBR322 region (Lusky and Botchan, ibid.). Plasmids pD1 (6.6 μg) and pML-1 Lusky and Botchan, ibid.) (4 μg) were incubated in 50 μl buffer A with 10 units each Eco RI and Nru I for 2 hours at 37°C, followed by agarose gel electrophoresis. The 1.7 kb pD1 fragment and 1.8 kb PML-1 fragment were isolated and ligated together (50 ng each) in 20 μl buffer C containing 100 units T4 polynucleotide ligase for 2 hours at 12°C, followed by transformation into E. coli HB101. Colonies containing the desired construct (designated ppD1) were identified by rapid preparation analysis. Ten μg of ppD1 was then digested with 20 units each Eco RI and Bgl II in 50 μl buffer A for 2 hours at 37°C. The DNA was electrophoresed through agarose, and the desired 2.8 kb fragment (fragment C) comprising the PML-1, 3′ splice site and poly A sequences was isolated.

To generate the remaining fragments used in constructing pD5, pDHFRIII was modified to convert the Sac II (Sst II) site into either a Hind III or Kpn I site. Ten μg pDHFRIII were digested with 20 units Sst II for 2 hours at 37°C, followed by phenol extraction and ethanol precipitation. Resuspended DNA was incubated in 100 μl buffer B containing 10 mM dCTP and 16 units T4 DNA polymerase for 60 minutes at 12°C, phenol extracted, dialyzed, and ethanol precipitated. DNA (5 μg) was ligated with 50 ng kinased Hind III or Kpn I linkers in 20 μl buffer C containing 400 units T4 DNA ligase for 10 hours at 12°C, phenol extracted, and ethanol precipitated. After resuspension in 50 μl buffer A, the resultant plasmids were digested with 50 units Hind III or Kpn I, as appropriate, and electrophoresed through agarose. Gel-isolated DNA (250 ng) was ligated in 30 μl buffer C containing 400 units T4 DNA ligase for 4 hours at 12°C and used to transform E. coli RR1. The resultant plasmids were designated pDHFRIII (Hind III) and pDHFRIII (Kpn I). A 0.4 kb Eco RI-Kpn I fragment (fragment A) was then purified from pDHFRIII (Kpn I) by digestion with Eco RI and Kpn I followed by agarose gel electrophoresis.

The SV40 enhancer sequence was inserted into pDHFRIII (Hind III) as follows: 50 μg SV40 DNA was incubated in 120 μl buffer A with 50 units Hind III for 2 hours at 37°C, and the Hind III C SV40 fragment (5171-1046 bp) was gel purified. Plasmid pDHFRIII (Hind III) (10 μg) was treated with 250 ng calf intestinal phosphatase for 1 hour at 37°C, phenol extracted and ethanol precipitated. The linearized plasmid (50 ng) was ligated with 250 ng Hind III C SV40 fragment in 16 μl buffer C for 3 hours at 12°C, using 200 units T4 polynucleotide ligase, and transformed into E. coli HB101. A 0.9 kb Kpn I-Bgl II fragment (fragment B) was then isolated from this plasmid.

For the final construction of pD5, fragments A and B (50 ng each) and 10 ng fragment C were ligated with 200 units T4 polynucleotide ligase for 4 hours at 12°C, and the mixture was transfected into E. coli RR1. Positive colonies were detected by rapid preparation analysis, and a large-scale preparation of pD5 (Figure 8) was made.

## C. Construction of Expression Vector p594

The expression of protein C cDNA was achieved in the vector pDX. This vector was derived from pD11 and pD5′. Plasmid pD5′ is identical to pD5 except that the SV40 polyadenylation signal (i.e., the SV40 Bam HI [2533 bp] to Bcl I [2770 bp] fragment) is in the late orientation. Thus, pD5′ contains a Bam HI site as the site of gene insertion. Plasmid pD11 differs from pD5 in that the Hind III (5171 bp in the SV40 genome) to Kpn I (294 bp in SV40) fragment, containing enhancer sequences, is in the opposite orientation.

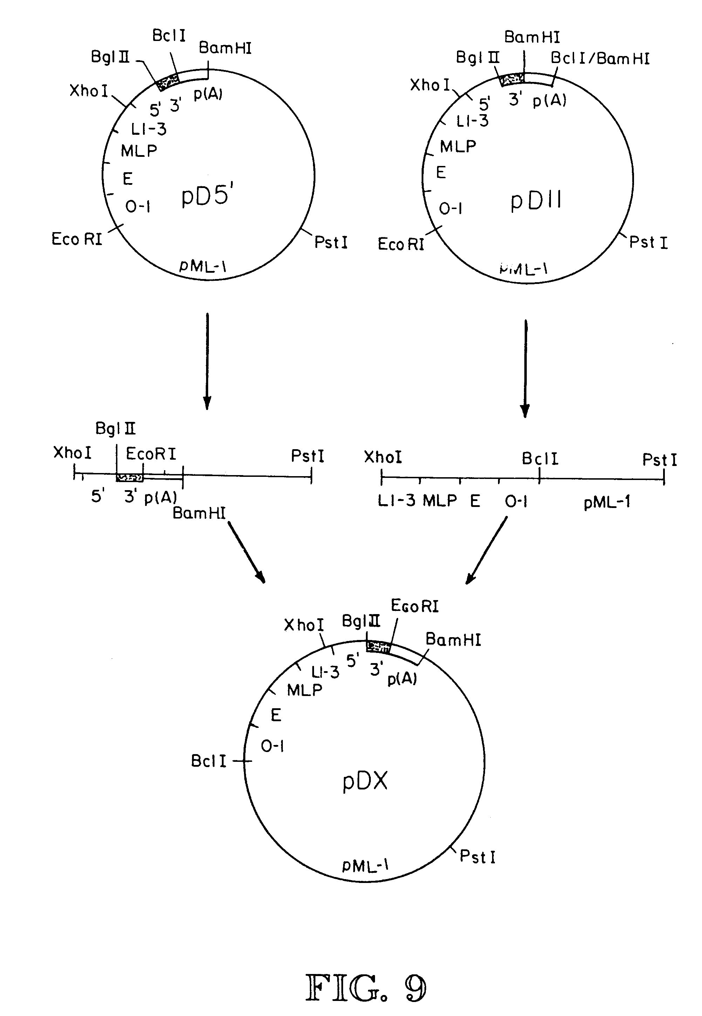

To generate pDX, the Eco RI site in pD11 was converted to a Bc1 I site by Eco RI cleavage, incubation with S1 nuclease, and subsequent ligation with Bcl I linkers. DNA was prepared from a positively identified colony, and the 1.9 kb Xho I-Pst I fragment containing the altered restriction site was prepared via agarose gel electrophoresis. In a second modification, Bcl I-cleaved pD5′ was ligated with kinased Eco RI-Bcl I adaptors (constructed from oligonucleotides ZC525, 5′ GGAATTCT 3′; and ZC526, 5′ GATCAGAATTCC 3′) in order to generate an Eco RI site as the position for inserting a gene into the expression vector. Positive colonies were identified by restriction endonuclease analysis, and DNA from this was used to isolate a 2.3 kb Xho I-Pst I fragment containing the modified restriction site. The two above-described DNA fragments were incubated together with T4 DNA ligase and transformed into E. coli HB101, and positive colonies were identified by restriction analysis. A preparation of such DNA, termed pDX (Figure 9), was then made. This plasmid contains a unique Eco RI site for insertion of foreign genes.

The protein C cDNA was then inserted into pDX as an Eco RI fragment. Recombinant plasmids were screened by restriction analysis to identify those having the protein C insert in the correct orientation with respect to the promoter elements, and plasmid DNA (designated pDX/PC) was prepared from a correct clone. Because the cDNA insert in pDX/PC contains an ATG codon in the 5′ non-coding region, deletion mutagenesis was performed on the cDNA prior to transfection and expression experiments. Deletion of the three base pairs was performed according to standard procedures of oligonucleotide-directed mutagenesis. The pDX-based vector containing the modified cDNA was designated p594.

### D. Expression of Protein C in a KEX2 Transfected Cell Line

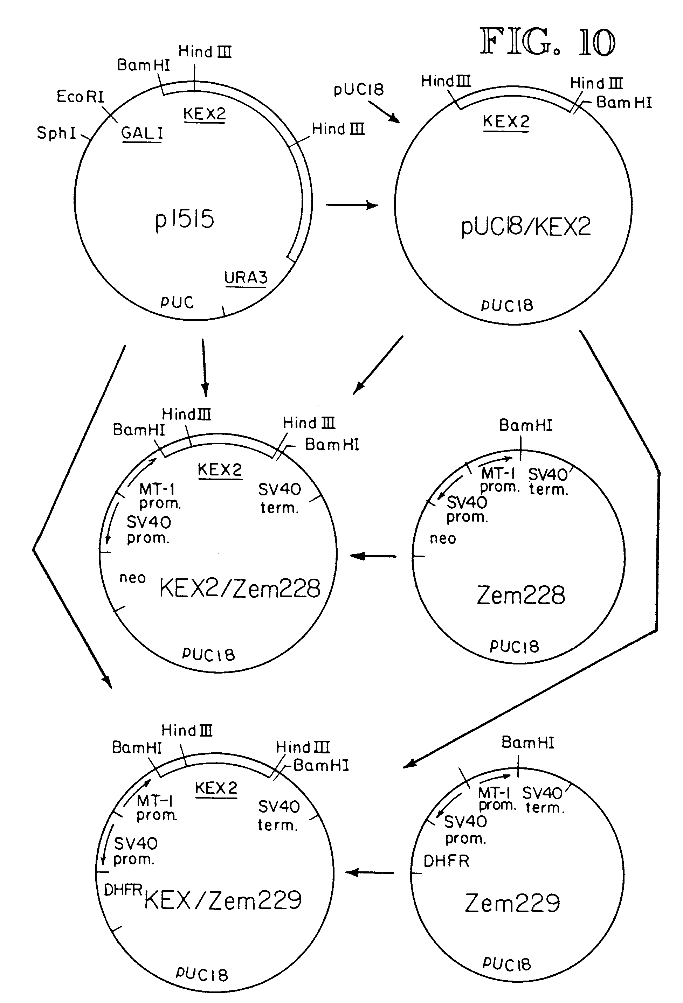

The Saccharomyces cerevisiae KEX2 gene was isolated from a yeast genomic library by screening transformed kex2 mutant cells for production of an α-factor halo on a lawn of suitable tester cells. One clone was obtained which complemented all reported defects of kex2 mutations (mating, α-factor production, maturation of killer toxin and sporulation in a kex2 homozygous diploid strain). The gene was subcloned into a pUC vector under the control of the yeast GAL1 promoter. The resultant plasmid, designated p1515, has been deposited with American Type Culture Collection as an E. coli HB101 transformant under accession number 67569. As shown in Figure 10, p1515 was digested with Hind III and a 2.1 kb fragment was recovered. This fragment was ligated to Hind III-cut pUC18 to construct plasmid pUC18/KEX2. The KEX2 fragment (2.1 kb) was then isolated from pUC18/KEX2 by digesting the plasmid partially with Hind III and to completion with Bam HI. The remainder of the KEX2 sequence was then isolated as a 0.43 kb fragment from a Bam HI + Hind III digest of p1515. The two KEX2 fragments were then ligated into the Bam HI site of the vectors Zem228 and Zem229 (Figure 10). (Zem229 is similar to Zem228 but contains a DHFR gene in place of the neomycin resistance gene. A cloning site is flanked by the MT-1 promoter and SV40 terminator.) The resulting plasmids were designated KEX2/Zem228 and KEX2/Zem229, respectively.

The BHK cell line tk⁻ts13 was co-transfected with the plasmids p594 and pSV2-DHFR by the calcium phosphate procedure. Transfected cells were selected with 250 nM methotrexate (MTX) and clonal cell lines were isolated. A clonal cell line which secreted protein C into the culture medium at 1.5 pg/cell/day was selected and designated PC594-204/BHK.

Ten μg of KEX2/Zem228 was transfected into the PC594-204/BHK cell line by the calcium phosphate procedure. Cells were cultured in the presence of 250 nM MTX at all times. Clones were selected with 500 μg/ml G418 and twelve clonal cell lines were selected.

The selected clones were pulse-labeled with ³⁵S-cysteine in cysteine-free MEM (Gibco) containing 1% fetal calf serum for 24 hours. The culture media were collected and examined for the presence of single-chain and cleaved two-chain protein C by immunoprecipitation with a monoclonal antibody to protein C. 250 μl of media was combined with 10 μg of the antibody and the mixture was incubated at 37°C for one hour. 100 μl of Staph A cell suspension (Pharmacia, Piscataway, NJ) was added and incubation was continued at 37°C for one hour. The cells were pelleted by centrifugation and the pellet was resuspended in TBS. The cells were again pelleted and the pellet was resuspended in 60 μl of gel buffer containing 1% β-mercaptoethanol. The suspension was heated to 100°C for three minutes, then electrophoresed on an SDS-polyacrylamide gel. Proteins were visualized by autoradiography. The parent cell line, PC594-204/BHK, showed approximately 70% of the protein C in the one-chain form, with the remaining 30% in the two-chain form. One of the G418-selected KEX2 cell lines, designated PC594-204/KEX2-1, produced 95% two-chain protein C, with the remaining 5% in the one-chain form.

### E. Modification of the Protein C Processing Site

To enhance the processing of single-chain protein C to the two-chain form, two additional arginine residues were introduced into the protein, resulting in a cleavage site consisting of four basic amino acids. The resultant mutant precursor of protein C was designated PC962. It contains the sequence Ser-His-Leu-Arg-Arg-Lys-Arg-Asp at the cleavage site between the light and heavy chains (Table 3). Processing at the Arg-Asp bond results in a two-chain protein C molecule.

The mutant molecule was generated by altering the cloned cDNA by site-specific mutagenesis (essentially as described by Zoller and Smith, DNA 3:479-488, 1984) using the mutagenic oligonucleotide ZC962 (5′ AGT CAC CTG AGA AGA AAA, CGA GAC A 3′). Plasmid p594 was digested with Sst I, the approximately 87 bp fragment was cloned into M13mp11, and single-stranded template DNA was isolated.

Following mutagenesis, a correct clone was identified by sequencing. Replicative form DNA was isolated and digested with Sst I to isolate the mutagenized fragment, which was joined with Sst I-cut p594 in a two part ligation. Clones having the Sst I fragment inserted in the desired orientation were identified by restriction enzyme mapping. The resulting expression vector was designated pDX/PC962.

Plasmid pDX/PC962 was co-transfected into tk⁻ ts13 BHK cells with pSV2-DHFR (Subramani et al., Mol. Cell. Biol. 1:854-864, 1981) by the calcium phosphate procedure (essentially as described by Graham and van der Eb, ibid.). The transfected cells were grown in Dulbecco's modified Eagle's medium (MEM) containing 10% fetal calf serum, 1x PSN antibiotic mix (Gibco 600-5640), 2.0 mM L-glutamine and vitamin K (5 $\mu$g/ml). The cells were selected in 250 nM methotrexate (MTX) for 14 days, and the resulting colonies were screened by the immunofilter assay (McCracken and Brown, BioTechniques, 82-87, March/April 1984). Plates were rinsed with PBS or No Serum medium (Dulbecco's plus penicillin-streptomycin, 5 $\mu$g/ml vitamin K). Teflon® mesh (Spectrum Medical Industries, Los Angeles, CA) was then placed over the cells. Nitrocellulose filters were wetted with PBS or No Serum medium, as appropriate, and placed over the mesh. After four hours incubation at 37°C, filters were removed and placed in 50 mM Tris pH 7.4, 5 mM EDTA, 0.05% NP-40, 150 mM NaCl, 0.25% gelatin for 30 minutes at room temperature. The filters were incubated for 1 hour at room temperature, with shaking, in biotin-labeled sheep polyclonal antibody to protein C, 1 $\mu$g/ml in the same buffer. Filters were then washed in the buffer and incubated 1 hour at room temperature, with shaking, in avidin-conjugated horseradish peroxidase (Boehringer-Mannheim), 1:1000 in the same buffer. Filters were washed in 50 mM Tris-HCl, pH 7.4, 5 mM EDTA, 1 mM NaCl, 0.25% gelatin, 0.4% Sarkosyl, 0.05% NP40, then in $H_2O$, and incubated in color reagent (60 mg HRP color development reagent [Bio-Rad], 20 ml methanol, 100 $\mu$l $H_2O_2$ in 100 ml 50 mM Tris pH 7.4, 150 mM NaCl). The reaction was stopped by transferring the filters to $H_2O$. Six of the most intensely reacting colonies were picked by cylinder cloning and grown individually in 10 cm plates.

Protein C production levels from nearly confluent cultures were measured by enzyme-linked immunosorbent assay (ELISA). Affinity purified polyclonal or heavy chain-specific monoclonal antibody to human protein C (100 $\mu$l of a 1 $\mu$g/ml solution in 0.1 M $Na_2CO_3$, pH 9.6), was added to each well of 96 well microtiter plates and the plates were incubated overnight at 4°C. The wells were then washed three times with PBS (5 mM phosphate buffer, pH 7.5, 0.15 M NaCl) containing 0.05% Tween-20 and then incubated with 100 $\mu$l of 1% bovine serum albumin, 0.05% Tween-20 in PBS at 4°C overnight. The plates were then rinsed several times with PBS, air dried, and stored at 4°C. To assay the samples, 100 $\mu$l of each sample was incubated for 1 hour at 37°C in the coated wells and the wells were rinsed with 0.05% Tween-20 in PBS. The plates were then incubated for 1 hour at 37°C with a biotin-conjugated, affinity-purified, sheep polyclonal antibody to protein C (30 ng/ml) in PBS containing 1% bovine serum albumin and 0.05% Tween-20. The wells were rinsed with PBS and incubated again for 1 hour at 37°C with avidin-conjugated alkaline phosphatase in PBS containing 1% bovine serum albumin and 0.05% Tween-20. The wells were then rinsed with PBS, and alkaline phosphatase activity was measured by the addition of 100 $\mu$l of phosphatase substrate (Sigma 104; 600 $\mu$g/ml) in 10% diethanolamine, pH 9.8, containing 0.3 mM $MgCl_2$. The absorbance at 405 nm was read on a microtiter plate reader. Results are given in Table 1.

TABLE 1

| Clone | Cell Number (x $10^{-7}$) | ELISA ng/ml | pg/cell/day |
|-------|---------------------------|-------------|-------------|
| 962-1 | 1.1 | 2500 | 2.20 |
| -2 | 0.8 | 1250 | 1.56 |
| -3 | 1.2 | 1350 | 1.12 |
| -4 | 1.2 | 550 | 0.46 |
| -5 | 1.2 | 1550 | 1.30 |
| -6 | 1.2 | 950 | 0.80 |

The clone BHK/962-1 was grown in larger scale culture, and several hundred micrograms of protein C were purified by affinity chromatography on a column prepared by coupling 7 mg of polyclonal sheep antibody against human protein C to 2 grams of CNBr-activated Sepharose 4B (Pharmacia Inc., Piscataway, NJ). Cell culture medium was applied to the column, the column was washed with 100 ml TBS (50 mM Tris, pH 7.5, 150 mM NaCl), and the protein C was eluted with TBS containing 3 M KSCN or with pH 11.5 buffer (25 mM potassium phosphate, pH 11.5, 0.2 M NaCl, 2% Tween 80, 0.5% $NaN_3$). Western blot analysis demonstrated that the mutant protein C was approximately 95% in the two-chain form, compared to about 20% two-chain protein C obtained from BHK cells transfected with the native sequence.

Milligram quantities of protein C were purified from either stable BHK cell clones expressing the PC962 mutant protein or stable 293 cell clones expressing the wild-type protein C (p594 transfected cells) using a monoclonal antibody column specific for the calcium-induced conformation of protein C. Cell culture media were applied to the column in the presence of 5 mM $CaCl_2$. Protein C was eluted from the column with TBS containing 10 mM EDTA. The use of this purification method permitted purification of completely active protein C without exposure to denaturing conditions. The purified protein C was analyzed by SDS/PAGE followed by silver staining and was shown to be >95% pure.

The BHK-produced PC962 protein was assayed for its ability to be activated to a form which shows both amidolytic and anticoagulant activities. Affinity-purified protein samples were exhaustively dialyzed against TBS then activated by incubation at 37°C for 1 hour with 0.1 volume of 1 unit/ml Protac C (American Diagnostica). Amidolytic activity was measured by adding aliquots of the activation mixture to 100 $\mu$l of 1 mM protein C substrate (Spectrozyme PCa, American Diagnostica) in a microtiter well and measuring the change in $A_{405}$ over time using a microtiter plate reader. Anticoagulant activity of the activated protein C was assayed as described by Sugo et al. (ibid.). The affinity-purified PC962 protein was demonstrated to be fully active in both amidolytic and anticoagulant assays. Elution from the antibody column with pH 11.5 buffer was shown to yield a protein with higher activity than that obtained using 3 M KSCN elution.

Clonal cell lines from the pDX/PC962 transfection into BHK cells were isolated by a process of limiting dilution. One plate of MTX-selected colonies (approximately 300 colonies) was trypsinized, counted, and re-plated into microtiter wells at an average of 0.5 cell/well. These were grown up in selective media containing 250 nM MTX. About 50% of the wells contained colonies. Wells containing identifiable colonies (1-2 mm diameter) were assayed by ELISA for protein C level in the media. For this assay, fresh medium was added to all the wells, the plates were incubated for 75 minutes, then the medium was removed and assayed. Five colonies which gave 75-minute accumulations of greater than 50 ng/ml (corresponding to over 1000 ng/ml/day) were split into 10-cm plates for larger scale culture. Protein C production levels for these clones ranged from 1.1 to 2.8 pg/cell/day.

A second plasmid, designated PC229/962, was constructed by inserting the PC962 cDNA into plasmid Zem229. An Eco RI fragment containing the PC962 cDNA from pDX/PC962 was ligated, with Eco RI-Bam HI synthetic oligonucleotide adapters, to Zem229, which had been cut with Bam HI and treated with phosphatase. The resulting vector is PC229/962.

Plasmid PC229/962 was transfected into BHK cells by the calcium phosphate method. Cells were cultured in Dulbecco's MEM containing 10% fetal calf serum and 5 $\mu$g/ml vitamin K. The 48-hour transient expression level from this transfection was approximately 25 ng/ml. After 2 days, the transfected cells were split into selective media containing 250 nM MTX and cultured for an additional 14 days. Three plates from this transfection, containing approximately 200 colonies each, were screened by the immunofilter assay, and the 24 most intensely reacting colonies were picked by cylinder cloning. These were grown individually in 10-cm plates, and their protein C production levels were measured. Colonies producing between 1.1 and 2.3 pg/cell/day were used for the production of stable, protein C-producing cell lines.

Expression vector pDX/PC962 and plasmid pKO-neo were co-transfected by the calcium phosphate method into 293 cells. Transfected cells were split into media containing 500 $\mu$g/ml G418 after 48 hours. After 10 days in selective media, immunofilter assays were done, and two clones were picked by cylinder cloning. Protein C production was found to range from 1 to 2 pg/cell/day. The cultures were scaled up, and protein C was purified by immuno-affinity chromatography. Greater than 95% of the protein C was found to be in the two-chain form.

The structure of the 962 mutant protein prepared from BHK and 293 cells was compared to that of wild-type protein C from 293 cells and from plasma. Analysis by SDS/PAGE followed by silver staining showed that all the recombinant proteins contained heavy and light chains which co-migrated with those of the plasma protein. The wild-type protein C synthesized in 293 cells contained a significant amount (approximately 20%) of single-chain, unprocessed protein of Mr = 66,000, whereas the mutant protein produced in either cell type was essentially completely processed to two chains. N-terminal sequence analysis showed that both the light and heavy chains of the recombinant wild-type and BHK/PC962 mutant proteins were properly processed. The extent of gamma carboxylation of the recombinant proteins was measured by two distinct ELISA systems. The first system recognizes both gamma-carboxylated and non-carboxylated forms of the protein, while the second utilizes specific antibodies which only recognize protein C which has undergone a gla-induced conformational change in the presence of calcium. Analysis indicated that approximately 60% of the recombinant protein C produced in BHK cells and 90%-95% of that produced in 293 cells was sufficiently gamma carboxylated to be recognized by the specific antibodies.

The three recombinant proteins were also analyzed for amidolytic and anticoagulant activity and the results were compared to the activity of plasma protein C. PC962 from BHK cells and wild-type protein C from 293 cells both showed full amidolytic activity. In the anticoagulant assay, protein C from BHK cells had essentially the same specific activity as plasma protein C, whereas both wild-type and PC962 mutant proteins from 293 cells consistently exhibited approximately 25% greater specific activity. One unit of protein C activity is defined as the amount in 1 ml of normal human plasma, which contains 4 $\mu$g of protein C per 1 ml (Gardiner and Griffin, Prog. Hematol. 13:265-278, 1983) specific activity = 250 units/mg). Wild-type protein C produced in 293 cells was consistently in excess of 300 units/mg.

## F. Expression of Activated Protein C

The cDNA sequence for protein C was altered by site-specific mutagenesis to delete the portion encoding the activation peptide. The altered sequence was then transfected into BHK and 293 cells and stably transfected cells were selected. Active protein C was detected in culture media samples from both cell lines.

To delete the activation peptide coding sequence, plasmid p594 was digested with Sst I and the ~880 bp fragment was purified and inserted into the Sst I site of M13mp10 (Messing, Methods Enzymol. 101:20-77, 1983). The 12 activation peptide codons were deleted by oligonucleotide-directed deletion mutagenesis (Zoller and Smith, DNA 3:479-488, 1984) using the mutagenic oligonucleotide ZC829 (5′ CTG AAA CGA CTC ATT GAT 3′). Replicative form DNA was prepared from mutant phage clones and digested with Sst I. The protein C fragment (~840 bp) was isolated and inserted into Sst I-digested p594. The resultant plasmids were screened for proper orientation of the Sst I fragment by restriction mapping using Bgl II. A correct plasmid was selected and designated pPC829. Plasmid pPC829 was sequenced to verify the presence of the desired coding sequence.

Plasmid pPC829 was co-transfected into tk⁻BHK cells (with plasmid PSVDHFRT (Lee et al., Nature 294:228-232, 1982)) and 293 cells (with pKO-neo (Southern and Berg, J. Mol. Appl. Genet. 1:327-341, 1982)) by calcium phosphate coprecipitation (Graham and van der Eb, ibid.). After 48 hours, culture media were harvested and assayed for protein C by ELISA. Results are shown in Table 2. At the same time, cultures were split 1:5 into media containing 500 $\mu$g/ml of G418 (293 cells) or 250 nM methotrexate (tk⁻BHK cells). After 10 days in the presence of selective media, stably transfected colonies were screened for protein C production by immunofilter assay.

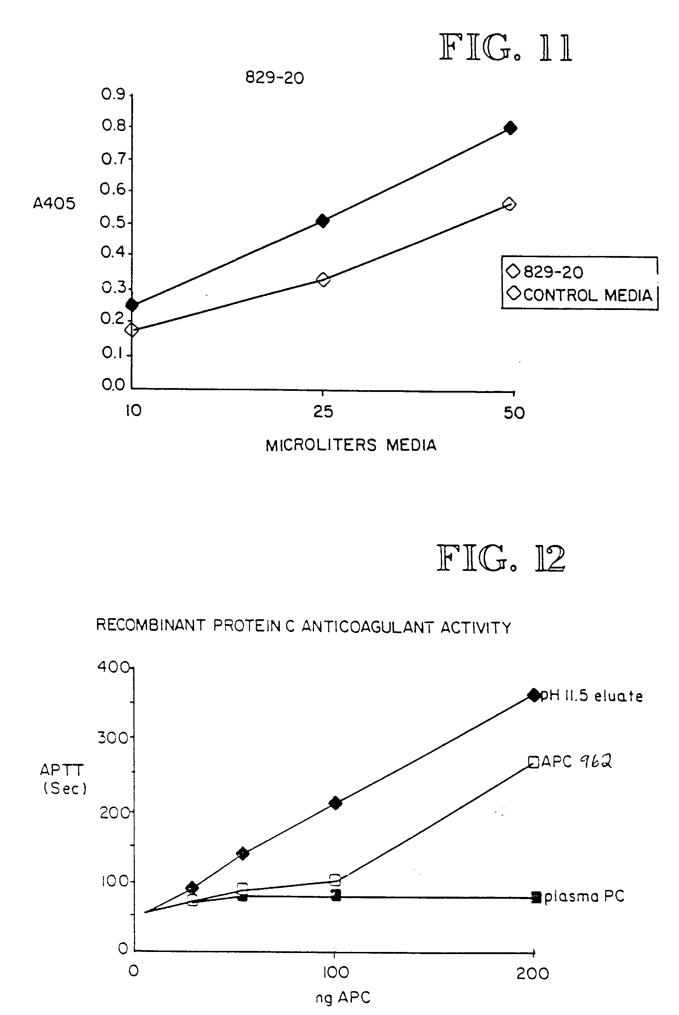

Positive colonies were picked and grown in selective media (containing 500 $\mu$g/ml G418 or 250 nM methotrexate, as appropriate) for 10 days. Culture media were assayed for APC activity by chromogenic assay. Media samples were added to microtiter wells containing 100 $\mu$l of 0.2 Spectrozyme PCa (American Diagnostica #336) in 50 mM Tris pH 7.5, 150 mM NaCl. Plates were incubated at 37°C, and the $A_{405}$ was measured at various time intervals. Representative results from one transfected 293 cell line (designated 829-20) are shown in Figure 11. Media from positive colonies of line 829-20 consistently showed higher activity with the chromogenic substrate for APC than did control media which had been incubated with non-transfected 293 cells for the same length of time (10 days).

TABLE 2

| TRANSIENT EXPRESSION OF ACTIVATED PROTEIN C (ELISA) | |
| --- | --- |
| Cell Line | Protein C ng/ml in Media |
| BHK | 2.7 |
| 293 | 30 |

A DNA sequence encoding an activated protein C precursor with the processing site sequence Arg-Arg-Lys-Arg was constructed by mutagenesis of the wild-type protein C sequence. The resultant sequence (designated 1058) was analogous to that encoding PC962, but lacked the portion encoding the activation peptide (Table 3).

The protein C sequence present in plasmid p594 was altered in a single mutagenesis to delete the codons for the activation peptide and insert the Arg-Arg codons at the processing site. Mutagenesis was performed on the 870 bp Sst I fragment from p594 essentially as described above using oligonucleotide ZC1058 (5′ CGC AGT CAC CTG AGA AGA AAA CGA CTC ATT GAT GGG 3′).

The mutagenized sequence was used to construct expression vector pDX/PC1058 (analogous to pDX/PC962) and the vector was co-transfected into BHK cells as described above. The protein was purified on a polyclonal antibody column eluted with pH 11.5 buffer.

The activity of the PC1058 protein was compared to that of activated plasma protein C and activated PC962. Plasma protein C and PC962 (5 $\mu$g/ml) were activated by treatment with 1/10 volume Protac C (American Diagnostica) for 2 hours. Anticoagulant activity was assayed by combining 50 $\mu$l human plasma with 50 $\mu$l activated protein C and incubating the mixtures at 37°C for 150 seconds. To this mixture was added 50 $\mu$l activated cephaloplastin (American Scientific Products, McGaw Park, IL), and the mixture was incubated at 37°C for 300 seconds. One hundred $\mu$l of 20 mM CaCl$_2$ was added and the clotting time was recorded. Data are presented in Figure 12.

## G. Expression of Activated Protein C and KEX2

A high protein C producing pDX/PC1058 transfected BHK clone (pDX/PC1058-3//BHK) was identified and transfected with KEX2/Zem229 by the calcium phosphate procedure. Transfected cells were selected with 500 $\mu$g/ml G418 and 250 nM methotrexate.

A selected clone designated KEX2-1058//BHK was pulse-labeled with $^{35}$S-cysteine in cysteine-free DMEM (Gibco) containing 1% fetal calf serum for 24 hours. The culture media were collected and examined for the presence of single-chain and cleaved, two-chain activated protein C by immunoprecipitation with a monoclonal antibody to protein C. Two hundred fifty $\mu$l of media was combined with 10 $\mu$g of antibody and the mixture was incubated at 37°C for one hour. One hundred $\mu$l of Staph A cell suspension (Pharmacia, Piscataway, NJ) was added and the mixture was incubated at 37°C for one hour. The cells were pelleted by centrifugation and the pellet was resuspended in 60 $\mu$l of gel buffer containing 1% $\beta$-mercaptoethanol. The suspension was heated to 100°C for three minutes, then electrophoresed on an SDS-polyacrylamide gel. Proteins were visualized by autoradiography. The KEX2-1058//BHK clone showed approximately 100% cleavage of the protein into the two-chain form.

Activated protein C was produced from the KEX2-1058//BHK clone grown to confluency in Dulbecco's MEM supplemented with 10% fetal calf serum, 250 nM methotrexate and 500 $\mu$g/ml G418. The confluent cells were switched to Dulbecco's MEM supplemented with 1 $\mu$g/ml fibronectin, 2 $\mu$g/ml insulin, 5 $\mu$g/ml transferrin, 5 $\mu$g/ml vitamin K, 1x PSN antibiotic mix (Gibco 600-5640), 2.0 mM L-glutamine, 250 nM methotrexate and 500 $\mu$g/ml G418. Media was collected every 1 to 2 days over a period of 7 days and was frozen at -20°. The frozen media samples were thawed and filtered through a 0.45 $\mu$m filter to remove any cell debris. Solid calcium chloride was added to a final concentration of 5 mM and solid sodium azide was added to a final concentration of 0.02% (W/V). Activated protein C was removed from the media using a monoclonal antibody column specific for the calcium-induced conformation of protein C. The treated media were applied to the column, and activated protein C was eluted with TBS containing 10 mM EDTA. Protein C concentration was determined by absorbance at 280 nm and by ELISA.

Protein C activity was measured by coagulation assay. Affinity purified plasma protein C was incubated with ACC-C (Agkistrodon contortrix contortrix protease [Kisiel et al., J. Biol. Chem. 262:12607-12613, 1987] obtained from W. Kisiel, University of New Mexico, Albuquerque, N.M.) diluted in 50 mM Tris, 100 mM NaCl and 0.1% bovine serum albumin at a ratio of 500:1 (APC:ACC-C) for 2 hours at 37°C. Affinity purified activated protein C from KEX2-1058//BHK cells was incubated for 2 hours at 37°C. Clot formation was measured in an MLA Electra 800 Coagulation Timer (Medical Laboratory Automation, Inc., Pleasantville, NY). One hundred $\mu$l of activated plasma protein C or KEX2-1058 activated protein C was added to an MLA cuvette and warmed for 50 seconds to raise the temperature to 37°C. One hundred $\mu$l of Dade Actin FS (American Scientific Products) was added and the test solutions were incubated for 100 sec. One hundred $\mu$l of 25 mM CaCl$_2$ was added to each cuvette. The time required for clot formation was measured. Results of coagulation assays showed that activated protein C produced by KEX2-1058//BHK cells is approximately 100% active relative to activated plasma protein C.

The carboxy-terminal sequence of the light chain of KEX2-1058 protein C was compared to the carboxy-terminal sequence of the light chain of commercially available protein C (American Diagnostica) using CNBr cleavage at the unique methionine residue of the light chain to liberate a peptide which could be sequenced in its entirety by N-terminal sequence analysis. Affinity purified protein C from KEX2-1058//BHK cells grown in Dulbecco's MEM supplemented with 1% fetal calf serum, 250 nM methotrexate and 500 $\mu$g/ml G418 was first reduced by the addition of a 10-fold molar excess (per Cys residue) of dithiothreitol (DTT) in 0.2 M Tris-HCl, pH 8.3, and guanidine-HCl to a final concentration of 6.0 M. The mixture was incubated at 65°C for 4-6 hours. Iodoacetic acid, pH 7.0, or iodoacetic amide was added to the reduced protein in a four-fold molar excess over the molar concentration of DTT, and the mixture was incubated for 30 minutes at 37°C. The solution was dialyzed against 0.1 M NH₄ HCO₃, pH 8.5 for 24 hours at 22°C. The dialyzed solution was applied to an HPLC Poly-F column (Dupont) to isolate the light chain. A 500-fold molar excess of CNBr per methionine residue was added to the purified light chain in 70% formic acid under nitrogen for 30 hours at room temperature in the dark. The CNBr digest was applied to an American Biosystems Inc. Model 470A sequenator (Marine-on-St. Croix, MN). The resultant sequences showed that the C-terminal sequence of both the commercially available purified protein C and the KEX2-1058 protein ended with Glu, indicating that the light chains of both proteins terminate at amino acid 149.

### H. Construction and Expression of p1645/Zem229R.

A DNA sequence encoding the amino acids Arg-Arg-Lys was substituted for the DNA sequence encoding amino acids 9-11 of the activation peptide encoded by the protein sequence of plasmid p962 (Table 3; the amino acids which have been added to the sequence encoding wild-type protein C appear in bold and spaces between amino acids are used solely for aligning the light and heavy chain sequences). Cells transfected with p1645 secreted activated protein C into the culture media. Plasmid p962 was digested with Sal I and Sst I and the 730 bp fragment was purified and inserted into M13mp10 which had been linearized by digestion with Sal I and Sst I. Synthetic oligonucleotides ZC1645 (5' GAA GAC CAA ACA ACA AAA CGG CTC ATT GAT 3') and ZC550 (5' TCC CAG TCA CGA CGT 3') were used to mutagenize the single-stranded template DNA prepared from the resultant phage by site-directed in vitro mutagenesis (Zoller and Smith, ibid.). The mutant phage were subjected to dideoxy-sequencing to confirm the mutagenesis. Replicative form (rf) DNA from a confirmed mutant clone, designated 1645, was prepared and was digested with Sst I and Pst I to isolate the 411 bp fragment. Plasmid p229/962 (Example 3.E.) was digested with Eco RI and Pst I to isolate the 592 bp protein C fragment. Plasmid p229/962 was also digested with Eco RI and Sst I to isolate the 700 bp protein C fragment. The 411 bp protein C fragment from the 1645 rf, the 411 bp protein C fragment from p229/962, and the 700 bp protein C fragment were joined in a four-part ligation with pZem229R which had been linearized with Eco RI and treated with calf intestinal phosphatase to prevent self-ligation. (Plasmid pZem229R is similar to Zem229 except that the Eco RI sites have been destroyed by partial digestion, blunt ending by Klenow fill, religation, subsequent digestion with Bam HI and religation with Bam HI-Eco RI adapters.) A correct plasmid was selected and was designated pPC1645/229R.

### Table 3

## Amino Acid Sequences of Cleavage-Site Mutants

|  |  | 155 |  | 170 |
|---|---|---|---|---|
| WT (594) | -S-H-L- | K-R-D-T-E-D-Q-E-D-Q-V-D-P-R- | | L-I-D-- |
| 829 | -S-H-L- | K-R- | | L-I-D-- |
| 962 | -S-H-L- | **R-R**-K-R-D-T-E-D-Q-E-D-Q-V-D-P-R- | | L-I-D-- |
| 1058 | -S-H-L- | **R-R-K-R-** | | L-I-D-- |
| 1645 | -S-H-L- | **R-R**-K-R-D-T-E-D-Q-E-D-Q-**R-R**-K-R- | | L-I-D-- |
| 1880 | -S-H-L- | **R-R**-K-R-D-T | D-Q-**R-R**-K-R- | L-I-D-- |
| 1953 | -S-H-L- | **R-R**-K-R- | **R-R**-K-R- | L-I-D-- |
| 1954 | -S-H-L- | **R-R**-K-R-D- | Q-**R-R**-K-R- | L-I-D-- |

Plasmid pPC1645/229R was transfected into tk⁻ BHK cells by calcium phosphate coprecipitation (Graham and van der Eb, ibid.). Transfected cells were subjected to selection with 1 μM methotrexate and media were assayed for protein C by ELISA (Example 3.E.). A positive clone was grown in Dulbecco's MEM supplemented with 10% fetal calf serum and 1 μM methotrexate until the cells reached confluency. The confluent cells were switched to Dulbecco's MEM supplemented with 1% fetal calf serum and 1 μM

methotrexate. Media were collected every 1 to 2 days over a period of 7 days and frozen at -20°C. The frozen media samples were thawed and filtered through a 0.45 $\mu$m filter to remove any cell debris. Solid calcium chloride was added to a final concentration of 5 mM and solid sodium azide was added to a final concentration of 0.02% (W/V). Protein C was recovered from the media using a monoclonal antibody column specific for the calcium-induced conformation of protein C. The treated media were applied to the column, and protein C was eluted with TBS containing 10 mM EDTA. Protein C concentration was determined by absorbance at 280 nm and by ELISA.

Activated protein C produced from pPC1645/229 transfected cells was compared to an equivalent amount of PC229/962 protein C produced from transfected cells using a chromogenic assay. One $\mu$g of affinity purified protein C diluted in 40 $\mu$l TBS + EDTA was added to each well of a 96-well plate. Forty $\mu$l of 2 mM Spectrozyme PCa (American Diagnostica Inc, New York, NY) was added to each well and incubated at 37°C until there was sufficient color development. Activity was measured as an increase in absorbance at 405 nm. The results showed that the activated protein C produced from pPC1645/229 transfected cells was 5-10% more active than the PC229/962-produced protein C.

### I. Construction and Expression of pPC1880/229R

The DNA sequence encoding protein C in plasmid 1645 was further modified to remove the first, second, seventh and eight amino acids of the activation peptide (Table 3). Single-stranded 1645 template DNA was prepared and was subjected to site-directed in vitro mutagenesis (Zoller and Smith, ibid.) using synthetic oligonucleotides ZC1880 (5'AAA CGA GAC ACA GAC CAA AGA AGA 3') and ZC550. Positive clones were subjected to dideoxy sequencing to confirm the mutagenesis. A positive clone was identified and was designated 1880.

Replicative form DNA prepared from clone 1880 was digested with Sst I and Pst I to isolate the 411 bp fragment. Plasmid PC229/962 was digested with Eco RI and Pst I to isolate the 562 bp protein C fragment. Plasmid PC229/962 was also digested with Eco RI and Pst I to isolate the 700 bp protein C fragment. The 411 bp protein C fragment from the 1880 rf and the 700 bp and 562 bp fragments from PC229/962 were joined with pZem229R, which had been digested with Eco RI, in a four-part ligation. A correct plasmid was selected and was designated pPC1880/229R.

Plasmid pPC1880/229R was transfected into tk⁻ BHK cells and assayed as previously described.

### J. Construction and Expression of pPC1954/229R

The coding sequence of the activation peptide present in plasmid 1645 is altered to remove the second through seventh amino acid codons of the activation peptide, resulting in a fusion between the first and eighth amino acid codons of the activation peptide present in 1645. Single-stranded 1645 template DNA is prepared and is subjected to site directed in vitro mutagenesis using the synthetic oligonucleotides ZC1954 (5' GAG AAG AAA ACG AGA CCA AAG AAG AAA AC 3') and ZC550. Positive clones are sequenced to confirm the mutagenesis. A positive clone is selected and is designated 1954. The amino acid sequence at the junction of the light and heavy chains of the encoded protein is shown in Table 3.

Replicative form DNA is prepared from clone 1954 and is digested with Sst I and Pst I to isolate the approximately 400 bp mutagenized protein C fragment. Plasmid PC229/962 is digested with Eco RI and Pst I and with Sst I and Eco RI to isolate the 562 bp Eco RI-Pst I fragment and the 700 bp protein C fragment, respectively. The approximately 400 bp protein C fragment from the 1954 rf and the 700 bp and 562 bp fragments from PC229/962 are joined with pZem229R, which has been digested with Eco RI, in a four-part ligation. A correct plasmid is selected and is designated pPC1954/229R.

Plasmid pPC1954/229R is transfected into tk⁻ BHK cells by calcium phosphate coprecipitation. Cells are selected and assayed as previously described.

### K. Construction and Expression of pPC1953/229R

The coding sequence of the activation peptide present in plasmid 1645 is altered to remove the first through eighth amino acid codons of the activation peptide, resulting in a fusion between the first and second sets of Arg-Arg-Lys-Arg amino acid codons present in 1645. Single-stranded 1645 template DNA is prepared and is subjected to site directed in vitro mutagenesis using the synthetic oligonucleotides ZC1953 (5' ACC TCA GAA GAA AAC GAA GAA GAA AAC GGC TCA T 3') and ZC550. Positive clones are sequenced to confirm the mutagenesis. A positive clone is selected and is designated 1953. The amino acid sequence at the light chain-heavy chain junction of the encoded protein is shown in Table 3.

Replicative form DNA is prepared from clone 1953 and is digested with Sst I and Pst I to isolate the approximately 400 bp mutagenized protein C fragment. Plasmid PC229/962 is digested with Eco RI and Pst I and with Sst I and Eco RI to isolate the 562 bp Eco RI-Pst I fragment and the 700 bp protein C fragment, respectively. The approximately 400 bp protein C fragment from the 1953 rf and the 700 bp and 562 bp fragments from PC229/962 are joined with pZem229R, which has been digested with Eco RI, in a four-part ligation. A correct plasmid is selected and is designated pPC1953/229R. Plasmid pPC1953/229R is transfected into tk⁻BHK cells by calcium phosphate coprecipitation. Cells are selected and assayed as previously described.

Example 4

Co-expression of Coagulation Factors VII and IX

Plasmid FIX/pD2 (Busby et al., Nature 316:271-273, 1985) was digested with Bam HI, and the 1.4 kb factor IX fragment was recovered. This fragment was then joined to pD5′ which had been digested with Bam HI and treated with calf intestinal phosphatase. The resultant plasmid was designated FIX/pD5′.

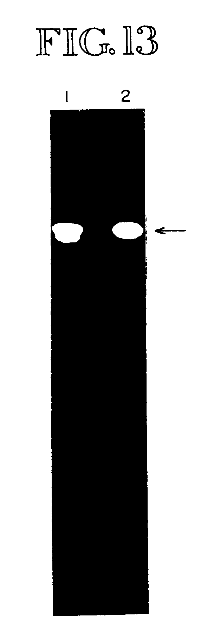

To co-express factor VII and factor IX, 10 μg of FIX/pD5′, 10 μg of FVII(565-2463)/pDX (Hagen et al., EP 200,421; ATCC 40205) and 1 μg of DHFRreS-pD5′ (a pD5′-derived plasmid containing a methotrexate resistant DHFR gene) [Levinson et al., EP 117,060]) were used to transfect tk⁻ BHK cells. Transfected cells were selected in the presence of methotrexate, then assayed for production of factor VII and factor IX by immunofilter assay using antibodies to both proteins. Colonies which were positive for both factor VII and factor IX production were selected, grown up and pulsed with ³⁵S-cysteine. Culture media and intracellular proteins were immunoprecipitated with the factor VII and IX antibodies and analyzed by polyacrylamide gel electrophoresis. Cells co-expressing factor VII and factor IX produced two-chain factor VII (i.e. factor VIIa). In contrast, cells producing only factor VII showed only the single chain form of the protein (Figure 13).

Example 5

Co-expression in Saccharomyces cerevisiae

The S. cerevisiae BAR1 gene encodes a secreted protein known as Barrier. The secretory peptide portion of the BAR1 gene product or the secretory peptide plus the third (C-terminal) domain of Barrier may be used to facilitate the secretion of foreign proteins produced in S. cerevisiae (MacKay, WO 87/02670 and MacKay et al., European Patent Application 88116335.6, both of which are incorporated herein by reference in their entirety).

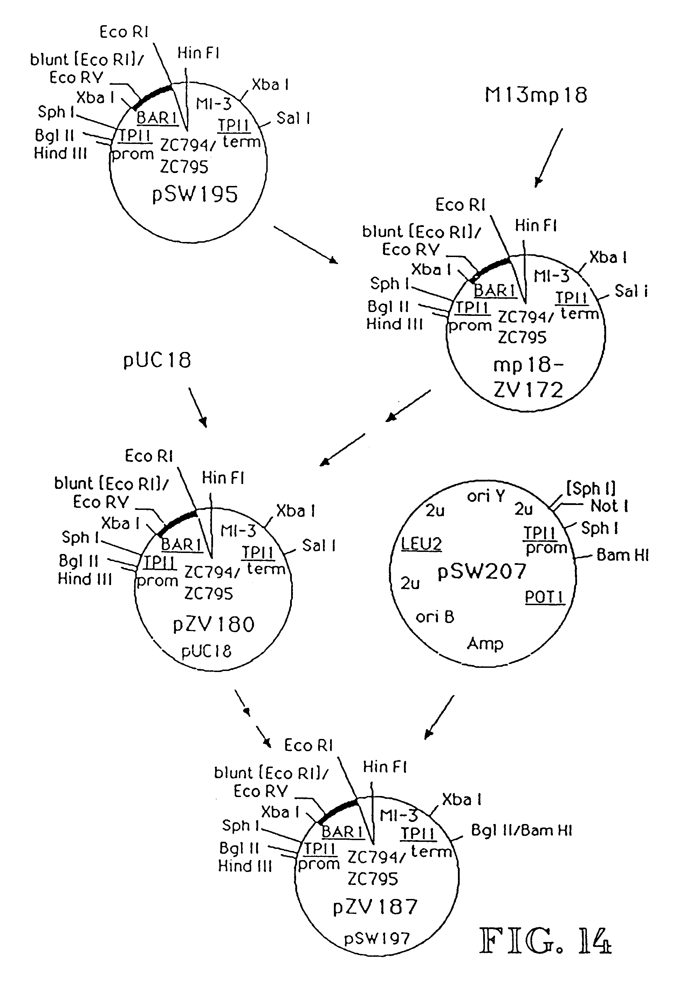

As described in European Patent Application 88116335.6, an expression vector containing the sequence encoding the signal peptide and third domain of Barrier fused to the coding sequence for the B(1-29)Ala-Ala-Lys-A(1-21) insulin precursor (Markussen et al., EP 163,529; hereinafter referred to as MI-3) was constructed. This vector, designated pSW195 (Figure 14), also contains the yeast TPI1 promoter and terminator. The Barrier and insulin sequences are joined at the amino acid sequence lys-arg. To allow processing of the fusion protein by thrombin, the lys-arg junction sequence was mutagenized to pro-arg. Cleavage at this site by thrombin will result in secretion of the unfused insulin precursor.

As shown in Figure 14, plasmid pSW195 was digested with Sph I and Sal I to isolate the 1.7 kb fragment comprising the BAR1-insulin fusion and the TPI1 terminator. This fragment was ligated with M13mp18 which had been previously digested to completion with Sph I and Sal I. The resultant phage clone was designated mp18-ZV172. Oligonucleotide ZC1083 (5′ TCC TTG GAT CCA AGA TTC GTT 3′) was used to mutagenize mp18-ZV172 using the uracil method (Kunkel, Proc. Nat'l Acad. Sci. USA 82:488-492, 1985). The resultant mutants were sequenced to confirm the mutagenesis and a positive clone was designated ZV172/1083. For convenience, the insert present in ZV172/1083 was subcloned into pUC18. The 1.7 kb Sph I-Sal I insert from ZV172/1083 was isolated and ligated with pUC18 which had been previously digested to completion with Sph I and Sal I. The resultant plasmid, pZV180, was digested to completion with Sal I. The adhesive ends of the linearized pZV180 were blunted using DNA polymerase I (Klenow fragment) and ligated to kinased Bgl II linkers. Excess linkers were removed by digestion with Bgl II. The linkered DNA was then cut to completion with Sph I to isolate the 1.7 kb insert. The 1.7 kb insert, comprising the partial TPI1 promoter, the BAR1-MI-3 fusion and the TPI1 terminator, was ligated into the Sph I-Bam HI partial TPI1 promoter-vector fragment of plasmid pSW207 to construct pZV187. (pSW207 was derived from pCPOT [ATCC 39685] by replacing the 750 bp Sph I-Bam HI fragment of pCPOT containing 2 micron and pBR322 sequences with a 186 bp Sph I-Bam HI fragment derived from the pBR322 tetracyline resistance gene; destroying the Sph I site and inserting a Not I site in the tetracycline resistance gene; digesting the resultant plasmid with Not I and Bam HI; and inserting a Not I-Bam HI TPI1 promoter fragment in place of the Not I-Bam HI pBR322-derived sequence.)

The thrombin cDNA is isolated from a prothrombin cDNA cloned in the Pst I site of pBR322 (Friezner-Degan et al., ibid.). The KEX2 gene from p1515 (Example 3) is digested and manipulated to remove the catalytic domain and the remaining sequences are joined to the thrombin cDNA. An expression unit is prepared by joining the hybrid gene to the TPI1 promoter and terminator. This expression unit is then substituted for the coding region of the yeast BAR1 gene. The resultant construct, comprising the expression unit flanked by BAR1 gene non-coding sequences, is used to transform S. cerevisiae. The transformed cells are cultured and screened for production of thrombin by enzyme activity assay. A colony positive for thrombin production is then transformed with pZV187). Cells are cultured and the insulin precursor is isolated from the media.

From the foregoing it will be appreciated that, although specific embodiments of the invention have been described herein for purposes of illustration, various modification may be made without deviating from the spirit and scope of the invention. Accordingly, the invention is not to be limited except as be the appended claims.

ATCC 20699, 39685, 53347 and 67569 are all deposits made under the Budapest Treaty and were made on 13 April 1984, 9 May 1984, 10 December 1985 and 3 December 1987, respectively.

United States Patent Application No. 053412 is available.

The features disclosed in the foregoing description, in the following claims and/or in the accompanying drawings may, both separately and in any combination thereof, be material for realising the invention in diverse forms thereof.

## Claims

1. A eukaryotic host cell transfected or transformed with a first DNA sequence encoding a protein of interest selected from t-PA, factor VII, factor IX, factor X, protein C, activated protein C, protein S, plasminogen, insulin and derivatives and analogs thereof. and at least one additional DNA sequence, said additional DNA sequence encoding a protein which processes or stabilizes the protein of interest.

2. The host cell of claim 1 wherein the eukaryotic host cell is a mammalian host cell or a yeast host cell.

3. The host cell of claim 1 wherein the additional DNA sequence is selected from sequences encoding proteases, protease inhibitors, and proteins which bind to the protein of interest.

4. The host cell of claim 1 wherein the first DNA sequence encodes factor VII and the additional DNA sequence encodes factor IX.

5. The host cell of claim 1 wherein the first DNA sequence encodes protein C or activated protein C and the additional DNA sequence is the yeast KEX2 gene.

6. The host cell of claim 1 wherein the first DNA sequence encodes t-PA and the additional DNA sequence encodes a protease inhibitor selected from TIMP, trypsin inhibitors and aprotinin.

7. The host cell of claim 1 wherein the first DNA sequence encodes plasminogen and the additional DNA sequence encodes alpha-1-antitrypsin or a variant thereof.

8. The host cell of claim 1 wherein the first DNA sequence encodes protein C and the additional DNA sequence encodes protein S.

9. The host cell of claim 1 wherein the first DNA sequence encodes factor VII and the additional DNA sequence encodes tissue factor.

10. A method for producing a protein of interest, comprising:
    culturing a host cell according to any of claims 1-9 under conditions which allow the first DNA sequence and the additional DNA sequence(s) to be expressed; and
    isolating the protein of interest from the host cell.

**11.** The method of claim 10 including, prior to the step of culturing, cotransfecting or cotransforming the host cell with multiple vectors, each containing a separate expression unit.

**12.** The method of claim 10 including, prior to the step of culturing, transfecting or transforming the host cell with a single vector containing multiple expression units.

**13.** The method of claim 10 including, prior to the step of culturing, transfecting the host cell with a single vector containing a single expression unit that is transcribed into a polycistronic message, wherein the host cell is a mammalian cell.

**14.** Mammalian cells transfected with an expression vector capable of integration in mammalian host cell DNA, the expression vector comprising a promoter operably linked to a DNA sequence which codes for a protein having substantially the same biological activity as human activated protein C, said sequence further coding for the amino acid sequence L-$R_1$-$R_2$-$R_3$-$R_4$-X-$R_5$-$R_6$-$R_7$-$R_8$-H, wherein L is essentially the light chain of activated protein C, $R_1$-$R_8$ are Lys or Arg, X is a peptide bond or a spacer peptide of 1-12 amino acids, and H is essentially the heavy chain of activated protein C, wherein said cells are further transfected with the KEX1 or KEX2 gene of Saccharomyces cerevisiae.

**Patentansprüche**

**1.** Eine eukaryontische Wirtszelle, die transfiziert oder transformiert mit einer ersten DNA-Sequenz ist, die ein interessierendes Protein kodiert, daß ausgewählt ist aus t-PA, Faktor VII, Faktor IX, Faktor X, Protein C, aktiviertem Protein C, Protein S, Plasminogen, Insulin und Derivaten und Analoga derselben, und wenigstens einer zusätzlichen DNA-Sequenz kodiert, wobei besagte zusätzliche DNA-Sequenz ein Protein, das das interessierende Protein verarbeitet oder stabilisiert.

**2.** Die Wirtszelle nach Anspruch 1, wobei die eukaryontische Wirtszelle eine Säugetier-Wirtszelle oder eine Hefe-Wirtszelle ist.

**3.** Die Wirtszelle nach Anspruch 1, wobei die zusätzliche DNA-Sequenz ausgewählt ist aus Sequenzen, die Proteasen, Protease-Inhibitoren und Proteine, die sich an das interessierende Protein binden, kodieren.

**4.** Die Wirtszelle nach Anspruch 1, wobei die erste DNA-Sequenz Faktor VII kodiert und die zusätzliche DNA-Sequenz Faktor IX kodiert.

**5.** Die Wirtszelle nach Anspruch 1, wobei die erste DNA-Sequenz Protein C oder aktiviertes Protein C kodiert und die zusätzliche DNA-Sequenz das Hefe-KEX2-Gen kodiert.

**6.** Die Wirtszelle nach Anspruch 1, wobei die erste DNA-Sequenz t-PA kodiert und die zusätzliche DNA-Sequenz einen Protease-Inhibitor kodiert, der ausgewählt ist aus TIMP, Trypsin-Inhibitoren und Aprotinin.

**7.** Die Wirtszelle nach Anspruch 1, wobei die erste DNA-Sequenz Plasminogen kodiert und die zusätzliche DNA-Sequenz alpha-1-Antitrypsin oder eine Variante desselben kodiert.

**8.** Die Wirtszelle nach Anspruch 1, wobei die erste DNA-Sequenz Protein C kodiert und die zusätzliche DNA-Sequenz Protein S kodiert.

**9.** Die Wirtszelle nach Anspruch 1, wobei die erste DNA-Sequenz Faktor VII kodiert und die zusätzliche DNA-Sequenz Gewebefaktor kodiert.

**10.** Ein Verfahren zur Herstellung eines interessierenden Proteins, welches umfaßt:
Kultivieren einer Wirtszelle nach einem der Ansprüche 1-9 unter Bedingungen, die es ermöglichen, daß die erste DNA-Sequenz und die zusätzliche(n) DNA-Sequenz(en) exprimiert werden; und
Isolieren des interessierenden Proteins aus der Wirtszelle.

**11.** Das Verfahren nach Anspruch 10, welches vor dem Kultivationsschritt Co-Transfektion oder Co-Transformation der Wirtszelle mit mehreren Vektoren, die jeder eine separate Expressionseinheit enthalten, einschließt.

**12.** Das Verfahren nach Anspruch 10, welches vor dem Kultivationsschritt Transfektion oder Transformation der Wirtszelle mit einem einzelnen Vektor, der mehrere Expressionseinheiten enthält, einschließt.

**13.** Das Verfahren nach Anspruch 10, welches vor dem Kultivationsschritt Transfektion der Wirtszelle mit einem einzelnen Vektor einschließt, der eine einzelne Expressionseinheit enthält, die in eine polycistronische Botschaft transkribiert wird, wobei die Wirtszelle eine Säugetierzelle ist.

**14.** Säugetierzellen, die mit einem Expressionsvektor transfiziert sind, der in der Lage ist zur Integration in Säugetier-Wirtszell-DNA, wobei der Expressionsvektor einen Promotor umfaßt, der operabel verknüpft ist mit einer DNA-Sequenz, die für ein Protein kodiert, das im wesentlichen dieselbe biologische Aktivität wie menschliches aktiviertes Protein C besitzt, wobei besagte Sequenz außerdem für die Aminosäuresequenz L-$R_1$-$R_2$-$R_3$-$R_4$-X-$R_5$-$R_6$-$R_7$-$R_8$-H, kodiert, worin L im wesentlichen die leichte Kette von aktiviertem Protein C ist, $R_1$-$R_8$ Lys oder Arg sind, X eine Peptidbindung oder ein Abstandspeptid mit 1-12 Aminosäuren ist und H im wesentlichen die schwere Kette von aktiviertem Protein C ist, wobei besagte Zellen außerdem mit dem KEX1- oder KEX2-Gen von Saccharomyces cerevisiae transfiziert sind.

**Revendications**

**1.** Cellule hôte eucaryotique transfectée ou transformée avec une première séquence d'ADN codant pour une protéine intéressante choisie entre le t-PA, le facteur VII, le facteur IX, le facteur X, la protéine C, la protéine C activée, la protéine S, le plasminogène, l'insuline et leurs dérivés et analogues, et au moins une séquence d'ADN supplémentaire, ladite séquence d'ADN supplémentaire codant pour une protéine provoquant la maturation ou la stabilisation de la protéine intéressante.

**2.** Cellule hôte suivant la revendication 1, ladite cellule hôte eucaryotique étant une cellule hôte de mammifère ou une cellule hôte de levure.

**3.** Cellule hôte suivant la revendication 1, dans laquelle la séquence d'ADN supplémentaire est choisie entre des séquences codant pour des protéases, des inhibiteurs de protéases et des protéines qui se lient à la protéine intéressante.

**4.** Cellule hôte suivant la revendication 1, dans laquelle la première séquence d'ADN code pour le facteur VII et la séquence d'ADN supplémentaire code pour le facteur IX.

**5.** Cellule hôte suivant la revendication 1, dans laquelle la première séquence d'ADN code pour la protéine C ou la protéine C activée et la séquence d'ADN supplémentaire est le gène KEX2 de levure.

**6.** Cellule hôte suivant la revendication 1, dans laquelle la première séquence d'ADN code pour le t-PA et la séquence d'ADN supplémentaire code pour un inhibiteur de protéase choisi entre le TIMP, des inhibiteurs de trypsine et l'aprotinine.

**7.** Cellule hôte suivant la revendication 1, dans laquelle la première séquence d'ADN code pour le plasminogène et la séquence d'ADN supplémentaire code pour l'alpha-1-antitrypsine ou un de ses variants.

**8.** Cellule hôte suivant la revendication 1, dans laquelle la première séquence d'ADN code pour la protéine C et la séquence d'ADN supplémentaire code pour la protéine S.

**9.** Cellule hôte suivant la revendication 1, dans laquelle la première séquence d'ADN code pour le facteur VII et la séquence d'ADN supplémentaire code pour un facteur tissulaire.

**10.** Procédé de production d'une protéine intéressante, comprenant :
la culture d'une cellule hôte suivant l'une quelconque des revendications 1 à 9 dans des conditions qui permettent l'expression de la première séquence d'ADN et de la ou des séquences d'ADN supplémentaires ; et

l'isolement de la protéine intéressante de la cellule hôte.

**11.** Procédé suivant la revendication 10, comprenant, avant l'étape de culture, la cotransfection ou la cotransformation de la cellule hôte avec des vecteurs multiples, chacun contenant une unité d'expression distincte.

**12.** Procédé suivant la revendication 10, comprenant, avant l'étape de culture, la transfection ou la transformation de la cellule hôte avec un seul vecteur contenant des unités d'expression multiples.

**13.** Procédé suivant la revendication 10, comprenant, avant l'étape de culture, la transfection de la cellule hôte avec un seul vecteur contenant une seule unité d'expression qui est transcrite en un message polycistronique, la cellule hôte étant une cellule de mammifère.

**14.** Cellules de mammifère transfectées avec un vecteur d'expression apte à l'intégration d'un ADN de cellule hôte de mammifère, le vecteur d'expression comprenant un promoteur lié de manière fonctionnelle à une séquence d'ADN qui code pour une protéine ayant pratiquement la même activité biologique que la protéine C activée humaine, ladite séquence codant en outre pour la séquence d'aminoacides $L-R_1-R_2-R_3-R_4-X-R_5-R_6-R_7-R_8-H$, dans laquelle L est essentiellement la chaîne légère de la protéine C activée, $R_1-R_8$ représentent Lys ou Arg, X représente une liaison peptidique ou un peptide intercalaire de 1 à 12 aminoacides, et H représente essentiellement la chaîne lourde de la protéine C activée, lesdites cellules étant en outre transfectées avec le gène KEX1 ou KEX2 de Saccharomyces cerevisiae.

FIG. 1

FIG. 2

# FIG. 3

FIG. 4

FIG. 5

# FIG.6

```
         10          20              36                      51
GCACTGCTGG CCAGTCCCAA A ATG GAA CAT AAG GAA GTG GTT CTT CTA CTT CTT TTA
                         MET Glu His Lys Glu Val Val Leu Leu Leu Leu Leu


           66                      81                  96                  111
TTT CTG AAA TCA GGT CAA GGA GAG CCT CTG GAT GAC TAT GTG AAT ACC CAG GGG
Phe Leu Lys Ser Gly Gln Gly Glu Pro Leu Asp Asp Tyr Val Asn Thr Gln Gly


                   126             141                 156
GCT TCA CTG TTC AGT GTC ACT AAG AAG CAG CTG GGA GCA GGA AGT ATA GAA GAA
Ala Ser Leu Phe Ser Val Thr Lys Lys Gln Leu Gly Ala Gly Ser Ile Glu Glu


       171                 186                 201                 216
TGT GCA GCA AAA TGT GAG GAG GAC GAA GAA TTC ACC TGC AGG GCA TTC CAA TAT
Cys Ala Ala Lys Cys Glu Glu Asp Glu Glu Phe Tyr Cys Arg Ala Phe Gln Tyr


CAC AGT AAA GAG CAA CAA TGT GTG ATA ATG GCT GAA AAC AGG AAG TCC TCC ATA
         231                 246                 261
His Ser Lys Glu Gln Gln Cys Val Ile MET Ala Glu Asn Arg Lys Ser Ser Ile


276                 291                 306                 321
ATC ATT AGG ATG AGA GAT GTA GTT TTA TTT GAA AAG AAA GTG TAT CTC TCA GAG
Ile Ile Arg MET Arg Asp Val Val Leu Phe Glu Lys Lys Val Tyr Leu Ser Glu


           336                 351                 366                 381
TGC AAG ACT GGG AAT GGA AAG AAC TAC AGA GGG ACG ATG TCC AAA ACA AAA AAT
Cys Lys Thr Gly Asn Gly Lys Asn Tyr Arg Gly Thr MET Ser Lys Thr Lys Asn


                   396             411                 426
GGC ATC ACC TGT CAA AAA TGG AGT TCC ACT TCT CCC CAC AGA CCT AGA TTC TCA
Gly Ile Thr Cys Gln Lys Trp Ser Ser Thr Ser Pro His Arg Pro Arg Phe Ser


       441                 456                 471                 486
CCT GCT ACA CAC CCC TCA GAG GGA CTG GAG GAG AAC TAC TGC AGA AAT CCA GAC
Pro Ala Thr His Pro Ser Glu Gly Leu Glu Glu Asn Tyr Cys Arg Asn Pro Asp


           501                 516                 531
AAC GAT CCG CAG GGG CCC TGG TGC TAT ACT ACT GAT CCA GAA AAG AGA TAT GAC
Asn Asp Pro Gln Gly Pro Trp Cys Tyr Thr Thr Asp Pro Glu Lys Arg Tyr Asp


546                 561                 576                 591
TAC TGC GAC ATT CTT GAG TGT GAA GAG GAA TGT ATG CAT TGC AGT GGA GAA AAC
Tyr Cys Asp Ile Leu Glu Cys Glu Glu Glu Cys MET His Cys Ser Gly Gly Asn


           606                 621                 636                 651
TAT GAC GGC AAA ATT TCC AAG ACC ATG TCT GGA CTG GAA TGC CAG GCC TGG GAC
Tyr Asp Gly Lys Ile Ser Lys Thr MET Ser Gly Leu Glu Cys Gln Ala Trp Asp


                   666                 681                 696
TCT CAG AGC CCA CAC GCT CAT GGA TAC ATT CCT TCC AAA TTT CCA AAC AAG AAC
Ser Gln Ser Pro His Ala His Gly Tyr Ile Pro Ser Cys Phe Pro Asn Lys Asn
```

31

# FIG. 6 CONT.

```
        771                    726                   741                   756
CTG AAG AAG AAT TAC TGT CGT AAC CCC GAG AGG GAG CTG CGC CCT TGG TGT TTC
Leu Lys Lys Asn Tyr Cys Arg Asn Pro Glu Arg Glu Leu Arg Pro Trp Cys Phe

                771                    786                   801
ACC ACC GAC CCC AAC AAG GCG TGG GAA CTT TGT GAC ATC CCC CGC TGC ACA ACA
Thr Thr Asp Pro Asn Lys Arg Trp Glu Leu Cys Asp Ile Pro Arg Cys Thr Thr

816                    831                   846                      861
CCT CCA CCA TCT TCT GGT CCC ACC TAC CAG TGT CTG AAG GGA ACA GCT GAA AAC
Pro Pro Pro Ser Ser Gly Pro Thr Tyr Gln Cys Leu Lys Gly Thr Gly Glu Asn

                876                    891                   906                   921
TAT CGC GGG AAT GTG GCT GTT ACC CTG TCC GGG CAC ACC TGT CAG CAC TGG AGT
Tyr Arg Gly Asn Val Ala Val Thr Val Ser Gly His Thr Cys Gln His Trp Ser

                936                    951                   966
GCA CAG ACC CCT CAC ACA CAT AAC AGG ACA CCA GAA AAC TTC CCC TGC AAA AAT
Ala Gln Thr Pro His Thr His Asn Arg Thr Pro Glu Asn Phe Pro Cys Lys Asn

    981                    996                   1011                  1026
TTG GAT GAA AAC TAC TGC CGC AAT CCT GAC GGA AAA AGG GCC CCA TGG TGC CAT
Leu Asp Glu Asn Tyr Cys Arg Asn Pro Asp Gly Lys Arg Ala Pro Trp Cys His

                1401                   1056                  1071
ACA ACC AAC AGC CAA GTG CGG TGG GAG TAC TGT AAG ATA CCG TCC TGT GAC TCC
Thr Thr Asn Ser Gln Val Arg Trp Glu Tyr Cys Lys Ile Pro Ser Cys Asp Ser

1086                   1101                  1116                  1131
TCC CCA GTA TCC ACG GAA CAA TTG GCT CCC ACA GCA CCA CCT GAG CTA ACC CCT
Ser Pro Val Ser Thr Glu Gln Leu Ala Pro Thr Ala Pro Pro Glu Leu Thr Pro

                1146                   1161                  1176                  1191
GTG GTC CAG GAC TGC TAC CAT GGT GAT GGA CAG AGC TAC CGA GGC ACA TCC TCC
Val Val Gln Asp Cys Tyr His Gly Asp Gly Gln Sre Tyr Arg Gly Thr Ser Ser

                1206                   1221                  1236
ACC ACC ACC ACA GSA AAG AAG TGT CAG TCT TGG TCA TCT ATG ACA CCA CAC CGG
Thr Thr Thr Thr Gly Lys Lys Cys Gln Ser Trp Ser Ser MET Thr Pro His Arg

    1251                   1266                  1281                  1296
CAC CAG AAG ACC CCA GAA AAC TAC CCA AAT GCT GGC CTG ACA ATG AAC TAC TGC
His Gln Lys Thr Pro Glu Asn Tyr Pro Asn Ala Gly Leu Thr MET Asn Tyr Cys

                1311                   1326                  1341
AGG AAT CCA GAT GCC GAT AAA GGC CCC TGG TGT TTT ACC ACA GAC CCC AGC GTC
Arg Asn Pro Asp Ala Asp Lys Gly Pro Trp Cys Phe Thr Thr Asp Pro Ser Val

1356                   1371                  1386                  1401
AGG TGG GAG TAC TGC AAC CTG AAA AAA TGC TCA GGA ACA GAA GCG AGT GTT GTA
Arg Trp Glu Tyr Cys Asn Leu Lys Lys Cys Ser Gly Thr Glu Ala Ser Val Val
```

32

# FIG. 6 CONT.

```
        1416                    1431                    1446                    1461
GCA CCT CCG CCT GTT GTC CTG CTT CCA GAT GTA GAG ACT CCT TCC GAA GAA GAC
Ala Pro Pro Pro Val Val Leu Leu Pro Asp Val Glu Thr Pro Ser Glu Glu Asp

            1476                    1491                    1506
TGT ATG TTT GGG AAT GGG AAA GGA TAC CGA GGC AAG AGG GCG ACC ACT GTT ACT
Cys MET Phe Gly Asn Gly Lys Gly Tyr Arg Gly Lys Arg Ala Thr Thr Val Thr

    1521                    1536                    1551                    1566
GGG ACG CCA TGC CAG GAC TGG GCT GCC CAG GAG CCC CAT AGA CAC AGC ATT TTC
Gly Thr Pro Cys Glu Asp Trp Ala Ala Gln Glu Pro His Arg His Ser Ile Phe

            1581                    1596                    1611
ACT CCA GAG ACA AAT CCA CGG GCG GGT CTG GAA AAA AAT TAC TGC CGT AAC CCT
Thr Pro Glu Thr Asn Pro Arg Ala Gly Leu Glu Lys Asn Tyr Cys Arg Asn Pro

1626                1641                    1656                    1671
GAT GGT GAT GTA GGT GGT CCC TGG TGC TAC ACG ACA AAT CCA AGA AAA CTT TAC
Asp Gly Asp Val Gly Gly Pro Trp Cys Tyr Thr Thr Asn Pro Arg Lys Leu Tyr

            1686                    1701                    1716                    1731
GAC TAC TGT GAT GTC CCT CAG TGT GCG GCC CCT TCA TTT GAT TGT GGG AAG CCT
Asp Tyr Cys Asp Val Pro Gln Cys Ala Ala Pro Ser Phe Asp Cys Gly Lys Pro

            1746                    1761                    1776
CAA GTG GAG CCG AAG AAA TGT CCT GGA AGG GTT GTA GGG GGG TGT GTG GCC CAC
Gln Val Glu Pro Lys Lys Cys Pro Gly Arg Val Val Gly Gly Cys Val Ala His

    1791                    1806                    1821                    1836
CCA CAT TCC TGG CCC TGG CAA GTC AGT CTT AGA ACA AGG TTT GGA ATG CAC TTC
Pro His Ser Trp Pro Trp Gln Val Ser Leu Arg Thr Arg Phe Gly MET His Phe

            1851                    1866                    1881
TCT GGA GGC ACC TTG ATA TCC CCA CAG TGG GTG TTG ACT GCT GCC CAC TGC TTG
Cys Gly Gly Thr Leu Ile Ser Pro Glu Trp Val Leu Thr Ala Ala His Cys Leu

1896                1911                    1926                    1941
GAG AAG TCC CCA AGG CCT TCA TCC TAC AAG GTC ATC CTG GGT GCA CAC CAA GAA
Glu Lys Ser Pro Arg Pro Ser Ser Tyr Lys Val Ile Leu Gly Ala His Gln Glu

            1956                    1971                    1986                    2001
GTG AAT CTC GAA CCG CAT GTT CAG GAA ATA GAA GTG TCT AGG CTG TTG TTG GAG
Val Asn Leu Glu Pro His Val Gln Glu Ile Glu Val Ser Arg Leu Phe Leu Glu

            2016                    2031                    2046
CCC ACA CGA AAA GAT ATT GCC TTG CTA AAG CTA AGC AGT CCT GCC GTC ATC ACT
Pro Thr Arg Lys Asp Ile Ala Leu Leu Lys Leu Ser Ser Pro Ala Val Ile Thr

    2061                    2076                    2091                    2106
GAC AAA GTA ATC CCA GCT TGT CTG CCA TCC CCA AAT TAT GTG GTC GCT GAC CGG
Asp Lys Val Ile Pro Ala Cys Leu Pro Ser Pro Asn Tyr Val Val Ala Asp Arg
```

33

# FIG. 6 CONT.

```
        2121                    2136                    2151
ACC GAA TGT TTC ATC ACT GGC TGG GGA GAA ACC CAA GCT ACT TTT GGA GCT GGC
Thr Glu Cys Phe Ile Thr Gly Trp Gly Glu Thr Gln Gly Thr Phe Gly Ala Gly

2166                    2181                    2196                    2211
CTT CTC AAG GAA GCC CAG CTC CCT GTG ATT GAG AAT AAA GTG TGC AAT CGC TAT
Leu Leu Lys Glu Ala Gln Leu Pro Val Ile Glu Asm Lys Val Cys Asn Arg Tyr

        2226                    2241                    2256                    2271
GAG TTT CTG AAT GGA AGA GTC CAA TCC ACC GAA CTC TGT GCT GGG CAT TTG GCC
Glu Phe Leu Asn Gly Arg Val Gln Ser Thr Glu Leu Cys Ala Gly His Leu Ala

            2286                    2301                    2316
GGA GGC ACT GAC AGT TGC CAG GGT GAC AGT GGA GGT CCT CTG GTT TGC TTC GAG
Gly Gly Thr Asp Ser Cys Gln Gly Asp Ser Gly Gly Pro Leu Val Cys Phe Glu

    2331                    2346                    2361                    2376
AAG GAC AAA TAC ATT TTA CAA GGA GTC ACT TCT TGG GGT CTT GGC TGT GCA CGC
Lys Asp Lys Tyr Ile Leu Gln Gly Val Thr Ser Trp Gly Leu Gly Cys Ala Arg

            2391                    2406                    2421
CCC AAT AAG CCT GGT GTC TAT GTT CGT GTT TCA AGG TTT GTT ACT TGG ATT GAG
Pro Asn Lys Pro Gly Val Tyr Val Arg Val Ser Arg Phe Val Thr Trp Ile Glu

2436                2451                    2464        2474        2484        2494
GGA GTG ATG AGA AAT AAT TAA TTGGACGGGA GACAGAGTGA CGCACTGACT CACCTAGAGG
Gly Val MET Arg Asn Asn

        2504 ·      2514        2524        2534        2544        2554        2564
CTGGAACGAG GGTAGGGATT TAGCATGCTG GAAATAACTG GCAGTAATCA AACGAAGACA CTGTCCCCAG


        2574        2584        2594        2604        2614        2624        2634
CTACCAGCTA CGCCAAACCT CGGCATTTTT TGTGTTATTT TCTGACTGCT GGATTCTGTA GTAAGGTGAC


    ·       2644        2654        2664        2674
ATAGCTATGA CATTTGTTAA AAATAAACTC TGTACTTAAC TTTGA
```

FIG. 7A          FIG. 7B

FIG. 8

FIG. 9

FIG. 10

FIG. 11

FIG. 12

RECOMBINANT PROTEIN C ANTICOAGULANT ACTIVITY

# FIG. 13

FIG. 14

# FIG. 15

STU I                                     AVA II

↓                                       ↓

TCTTTGGATAAAAGAAGGCCTGATTTCTGTTTGGAACCTCCATACACTGG

TCCATGTAAAGCTAGAATCATCAGATACTTCTACAACGCCAAGGCTGGTT

TGTGTCAAACTTTCGTTTACGGTGGCTGCAGAGCTAAGAGAAACAACTTC

AAGTCTGCTGAAGACTGCATGAGAACTTGTGGTGGTGCCTAATCTAG

↑

XBA I

EP 0 319 944 B1